# EUROPEAN PATENT APPLICATION

(11) **EP 4 821 512 A1**
(43) Date of publication of application: **14.10.2026**
(21) Application number: 25761180.6
(22) Date of filing: 27.04.2025
(51) Int. Cl.: A61K 51/08, A61K 51/04, A61K 38/04, A61K 103/00, C07K 14/00

(54) **RADIOLABELED COLLAGEN HYBRIDIZING PEPTIDE AND PREPARATION METHOD THEREFOR AND USE THEREOF**

(30) Priority: 28.02.2024 CN 202410219633; 09.04.2025 CN 202510438663
(71) Applicant: The Fifth Affiliated Hospital of Sun Yat-Sen University, Zhuhai, Guangdong 519000 (CN)
(72) Inventor: LI, Yang, Zhuhai, Guangdong 519000 (CN); ZHOU, Daoning, Zhuhai, Guangdong 519000 (CN); LIU, Yinghua, Zhuhai, Guangdong 519000 (CN); MA, Xiaotong, Zhuhai, Guangdong 519000 (CN); ZHAO, Suwen, Zhuhai, Guangdong 519000 (CN)
(74) Representative: Gassner, Birgitta
(86) International application number: PCT/CN2025/091462
(87) International publication number: WO 2025/180547

(57) **Abstract**

Disclosed in the present invention are a radiolabeled collagen hybridizing peptide and a preparation method therefor and the use thereof. The radiolabeled collagen hybridizing peptide of the present invention contains a radionuclide-chelator complex bound via a coordination bond, and a collagen hybridizing peptide. The radiolabeled collagen hybridizing peptide of the present invention has good stability, and an excellent definition and signal-to-noise ratio, can not only sensitively detect precancerous lesions of pancreatic ductal adenocarcinoma (e.g., pancreatic intraepithelial neoplasia), but can also reduce, after optimization, the uptake in non-target organs such as the liver and kidneys, thereby having good clinical application prospects.

## Description

### CROSS-REFERENCE TO RELATED APPLICATIONS

This application claims priority to Chinese patent application No. 2024102196335, filed on February 28, 2024, and Chinese patent application No. 202510438663X, filed on April 9, 2025, the entire disclosures of which are incorporated herein by reference.

### TECHNICAL FIELD

The present disclosure relates to the field of biomedical technology, and more particularly to a radiolabeled collagen hybridizing peptide (CHP), a preparation method therefor, and use thereof.

### BACKGROUND

Fibrosis is a common pathological feature in the development of various chronic diseases, essentially characterized by excessive deposition and abnormal distribution of the extracellular matrix (ECM) during the repair process following tissue injury. Studies have shown that chronic fibrosis is not only a risk factor for cancer, but the fibrotic matrix itself is also a significant characteristic of malignant tumors such as pancreatic ductal adenocarcinoma (PDAC), breast cancer, prostate cancer, and colorectal cancer. Hyperplastic fibrotic stroma can account for 90% or more of the tumor mass. This dense fibrotic barrier at the tumor site severely impedes drug penetration for killing tumor cells and also limits the detection capability of conventional molecular probes targeting tumor cells. Consequently, to enable earlier detection and more precise diagnosis of fibrotic diseases, the development of sensitive, stable molecular probes with low background signals has become an inevitable trend.

In related technologies, radionuclide molecular probes are primary tools for early diagnosis. Current clinical research is increasingly shifting the detection target from tumor cells to the disease microenvironment, such as the surrounding ECM, thereby converting the dense barrier that hinders probe penetration into an opportunity for target binding. A prominent example is the fibroblast activation protein inhibitor (FAPI), which targets tumor-associated fibroblasts and has been widely studied in clinical research. Various radionuclide-labeled FAPI probes have shown excellent uptake and imaging effects in multiple tumors and are widely used in clinical research for lung and myocardial fibrosis. As the most abundant protein in the fibrotic ECM, collagen is a particularly attractive target. The basic structural unit of collagen is the Gly-Xaa-Yaa sequence, where glycine (Gly, O) is the common initiating amino acid, and the Xaa and Yaa positions are typically occupied by proline (Pro, P) and hydroxyproline (Hyp, O), respectively. Three left-handed polyproline II (PPII) polypeptide chains, each composed of periodically repeating basic structural units, tightly intertwine via interchain hydrogen bonds to form a stable right-handed triple helix. In fibrotic lesions, collagen synthesis and metabolism within the tissue increase significantly. Under the degradative action of abnormally elevated collagenases (such as matrix metalloproteinases, MMPs), the triple-helical conformation of collagen unfolds, leaving a large amount of structurally denatured collagen chains in pathological tissues. Based on this specific triple-helix structure, collagen hybridizing peptide (CHP) composed of (GPO)n or the optimized probe (GfO)n sequences has been designed. This peptide possesses a propensity for triple-helix formation and can hybridize with incomplete or unfolded collagen chains after degradation to re-form a triple helix, thereby specifically labeling damaged and denatured collagen in pathologically injured tissues without interacting with intact structured collagen or other proteins lacking a triple-helix structure. Moreover, CHP exhibits high specificity and good in vivo stability, making it suitable for targeted imaging of fibrotic tissues and providing information on lesion sites. However, during practical application, currently used collagen hybridizing peptide probes still face challenges such as poor labeling stability, low signal-to-noise ratio (SNR), and suboptimal biodistribution based on hepatic and renal metabolism.

Therefore, there is an urgent need for a radiolabeled CHP with superior stability and a high SNR to improve early diagnosis of fibrosis-related diseases.

### SUMMARY

An objective of the first aspect of the present disclosure is to provide a radiolabeled CHP.

An objective of the second aspect of the present disclosure is to provide a method for preparing a radiolabeled CHP.

An objective of the third aspect of the present disclosure is to provide a conjugate.

An objective of the fourth aspect of the present disclosure is to provide an imaging agent.

An objective of the fifth aspect of the present disclosure is to provide use of the radiolabeled CHP in the preparation of a product for specifically targeting a structurally denatured collagen, in the preparation of a product for diagnosis or lesion imaging of cancer characterized by fibrotic lesions, in the preparation of a product for detecting an organ fibrotic disease, and in the evaluation or screening of an anti-pancreatic ductal adenocarcinoma drug or an anti-fibrotic drug.

An objective of the sixth aspect of the present disclosure is to provide a kit for imaging tumors or organ fibrosis.

An objective of the seventh aspect of the present disclosure is to provide a method for treating or diagnosing a disease associated with organ fibrosis.

To achieve the aforementioned objectives, the technical solution of the present disclosure is as follows:
In the first aspect, the present disclosure provides a radiolabeled collagen hybridizing peptide, comprising:
(a) a radionuclide-chelator complex bound via a coordination bond; and
(b) a collagen hybridizing peptide (CHP);

wherein the radionuclide-chelator complex and the CHP is connected through a linker, the linker comprises at least one selected from the group consisting of a lysine residue, an oligoglycine residue, 6-aminohexanoic acid (Ahx), and polyethylene glycol (PEG); and
an amino acid sequence of the CHP is (GfO)ₙ or (GPO)ₙ, where n is a positive integer between 6 and 10.

It is understood that in the (GfO)ₙ or (GPO)ₙ, G represents glycine, f represents fluorinated proline, O represents hydroxyproline, and P represents proline.

The CHP designed based on the specific triple-helix structure of collagen is composed of (GPO)n or the optimized probe (GfO)ₙ. It possesses a propensity for triple-helix formation and can hybridize with incomplete or unfolded collagen peptide chains after degradation to form a triple helix, thereby labeling damaged and denatured collagen in pathologically injured tissues, without interacting with intact structured collagen or other proteins lacking a triple-helix structure. CHP exhibits high specificity and good in vivo stability, making it suitable for detection and imaging of tissue collagen remodeling caused by disease or collagen destruction caused by mechanical injury, providing information on the site of damaged collagen.

In some embodiments of the present disclosure, the amino acid sequence of the CHP is (GfO)ₙ, where n is specifically 8, 9, or 10.

While both (GfO)ₙ and (GPO)ₙ are CHP that bind to denatured collagen through triple-helix hybridization, (GPO)n is prone to self-trimerization and must be heated to unwind before hybridizing with the denatured collagen during actual use. In contrast, (GfO)ₙ does not undergo self-trimerization and can be used directly without heating.

In some embodiments of the present disclosure, the radionuclide of the radionuclide-chelator complex is any one selected from the group consisting of Al¹⁸F, ⁶⁴Cu, ⁶⁷Cu, ⁶⁷Ga, ⁶⁸Ga, ⁹⁹mTc, ⁸⁹Zr, ¹¹¹In, ¹⁷⁷Lu, ¹⁸⁶Re, and ²²⁵Ac.

In some embodiments of the present disclosure, the radionuclide is any one selected from the group consisting of ⁶⁸Ga, ⁶⁴Cu, or Al¹⁸F.

In some more preferably embodiments of the present disclosure, the radionuclide is Al¹⁸F.

It was discovered that when the radionuclide is Al¹⁸F, the probe achieves superior in vivo visualization and early diagnosis of PDAC. In the early diagnosis of PDAC, detecting precancerous pancreatic intraepithelial neoplasia (PanIN) is the primary goal. However, PanIN lesions are extremely small with few targets, and the pancreas is adjacent to abdominal metabolic organs, such as liver, spleen, intestines, and kidneys. Probes using conventional radionuclides like ⁶⁸Ga and ⁶⁴Cu often suffer from unsuitable biodistribution, where signals from metabolic organs (liver, spleen, intestines, kidneys) obscure the precancerous lesion signals, thereby affecting the detection results. Through continuous optimization of the probe, the present disclosure has found that the optimized probe using Al¹⁸F exhibits optimal biodistribution and an excellent signal-to-noise ratio, enabling the detection of weak lesion signals.

In some embodiments of the present disclosure, the chelator of the radionuclide-chelator complex is any one selected from the group consisting of 1,4,7-triazacyclononane-1,4,7-triacetic acid (NOTA), 1,4,7-triazacyclononane-1-glutaric acid-4,7-diacetic acid (NODAGA), hydrazinonicotinic acid (HYNIC), 1,4,7,10-tetraazacyclododecane-1,4,7,10-tetraacetic acid (DOTA), diethylenetriaminepentaacetic acid (DTPA), and derivatives thereof.

In some embodiments of the present disclosure, the NOTA derivatives may include NOTA-NH₂ (also referred to as NOTA2) or p-SCN-Bn-NOTA. The NOTA-NH₂ has a structure represented by Formula (a-1): 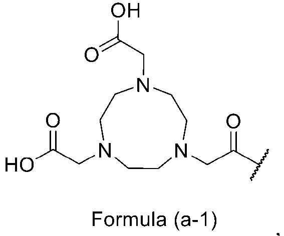 and the p-SCN-Bn-NOTA has a structure represented by Formula (a-2):

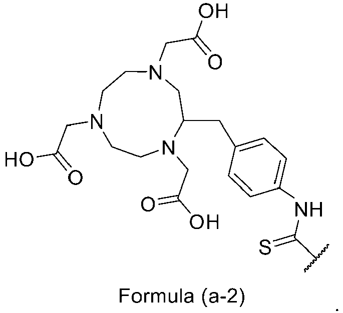

In some preferred embodiments of the present disclosure, when the radionuclide is ⁶⁸Ga, the chelator is the NOTA derivative represented by Formula (a-1), and the linker is 6-aminohexanoic acid, the radiolabeled collagen hybridizing peptide exhibits high uptake in target organs like tumors and low uptake in non-target organs like the liver and kidney, showing great clinical potential.

In some embodiments of the present disclosure, the NODAGA has a structure represented as follows:

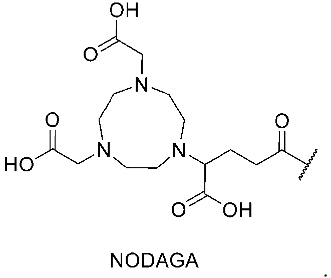

In some embodiments of the present disclosure, the DOTA has a structure represented as follows:

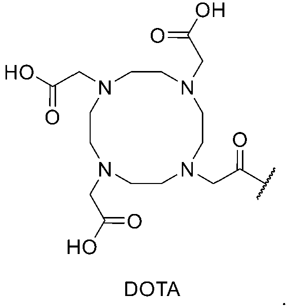

In some embodiments of the present disclosure, the radiolabeled CHP further comprises a fluorescent dye.

In some embodiments of the present disclosure, the fluorescent dye is connected to the linker through an amino acid or an amino acid derivative.

In some embodiments of the present disclosure, the fluorescent dye is at least one selected from the group consisting of a cyanine dye, rhodamine, 4,4-difluoro-4-bora-3a,4a-diaza-s-indacene (BODIPY), fluorescein isothiocyanate (FITC), erythrosin, phthalocyanine, phycocyanin, phycoerythrin, and an Alexa Fluor dye.

In some embodiments of the present disclosure, the cyanine dye is any one selected from the group consisting of sulfo-Cy3, sulfo-Cy5, sulfo-Cy5.5, sulfo-Cy7, and sulfo-Cy7.5.

In some preferred embodiments of the present disclosure, the cyanine dye is sulfo-Cy3 or sulfo-Cy5.

In some embodiments of the present disclosure, the rhodamine is at least one selected from the group consisting of 5-carboxy rhodamine, 6-carboxy rhodamine, 6-carboxytetramethylrhodamine, rhodamine isothiocyanate, and tetramethylrhodamine.

In some embodiments of the present disclosure, the BODIPY is at least one selected from the group consisting of BODIPY 630/650, BODIPY 650/665, BODIPY-FL, BODIPY-R6G, BODIPY-TMR, and BODIPY-TRX.

In some embodiments of the present disclosure, the phycocyanin is at least one selected from the group consisting of allophycocyanin, phycocyanin C, and phycocyanin R.

In some embodiments of the present disclosure, the Alexa Fluor dye is any one selected from the group consisting of Alexa Fluor 488, Alexa Fluor 555, Alexa Fluor 647, Alexa Fluor 700, and Alexa Fluor 750.

In some embodiments of the present disclosure, the amino acid derivative includes 6-aminohexanoic acid.

In some embodiments of the present disclosure, when the linker comprises a lysine residue and 6-aminohexanoic acid, a chemical structural formula of the radiolabeled CHP is as shown in Formulae (I): 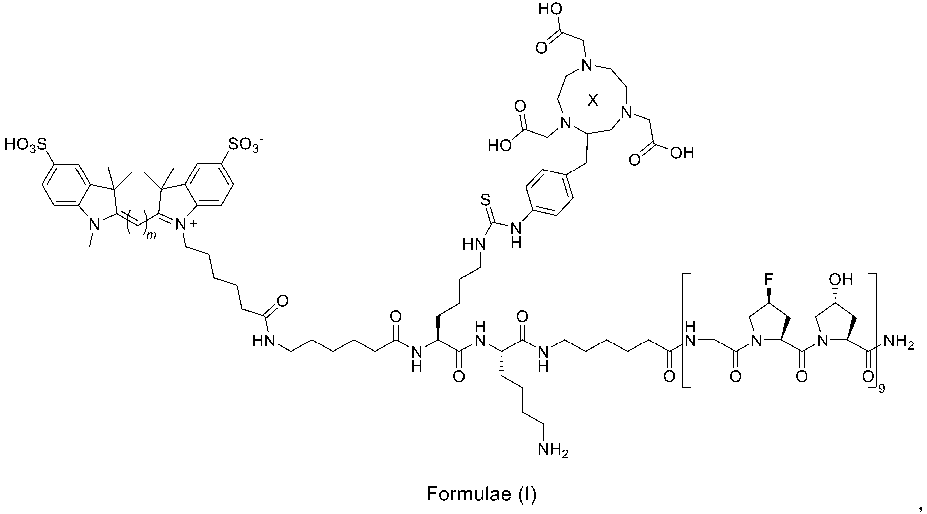 wherein, m is 3 or 5; X is a radionuclide which is any one selected from the group consisting of Al¹⁸F, ⁶⁴Cu, ⁶⁷Cu, ⁶⁷Ga, ⁶⁸Ga, ⁹⁹mTc, ⁸⁹Zr, ¹¹¹In, ¹⁷⁷Lu, ¹⁸⁶Re, and ²²⁵Ac.

In some embodiments of the present disclosure, when the linker comprises a lysine residue and 6-aminohexanoic acid, the radiolabeled CHP is a compound represented by any one of Formulae (I-1) to Formulae (I-4): 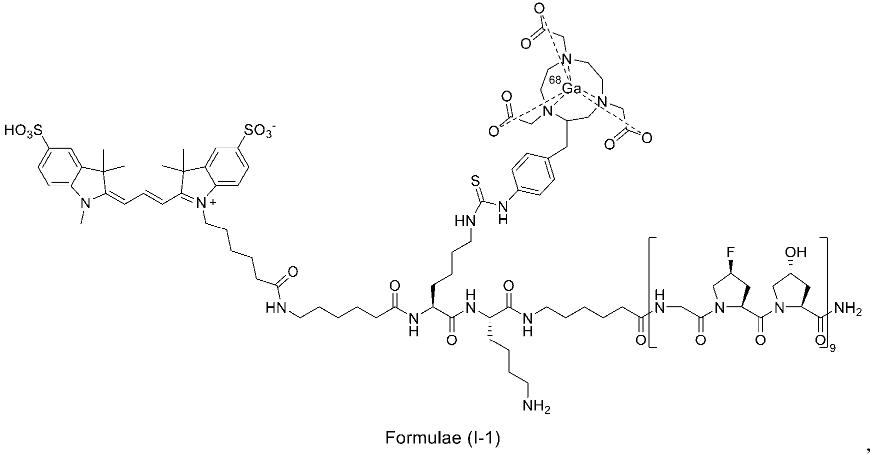 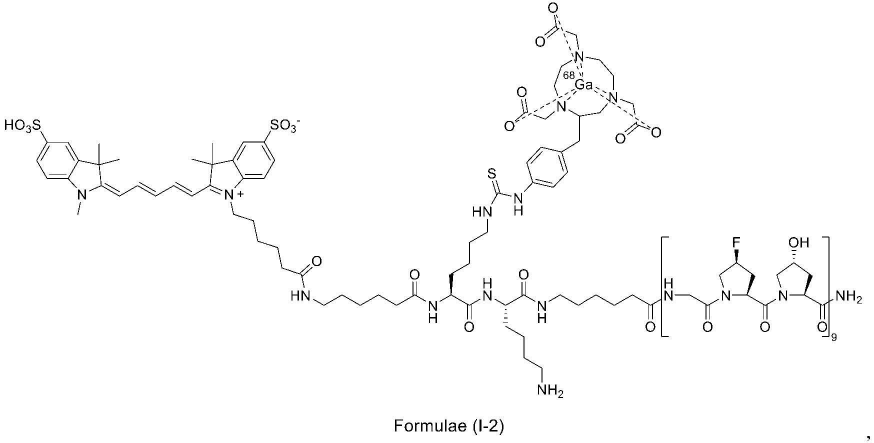 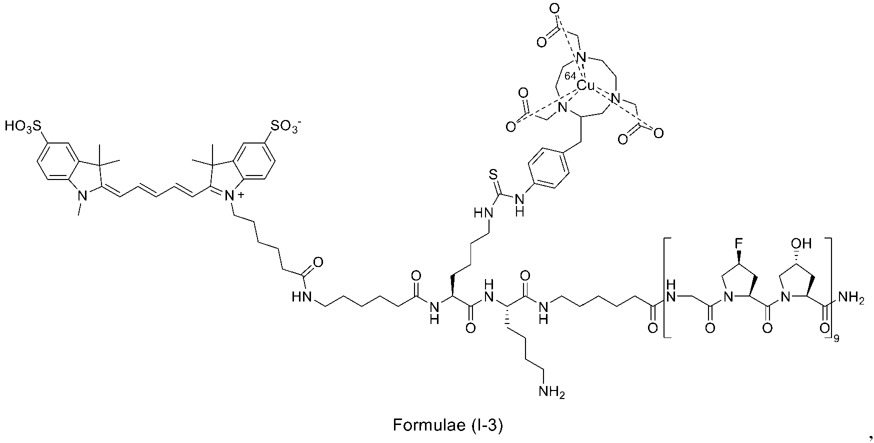 and

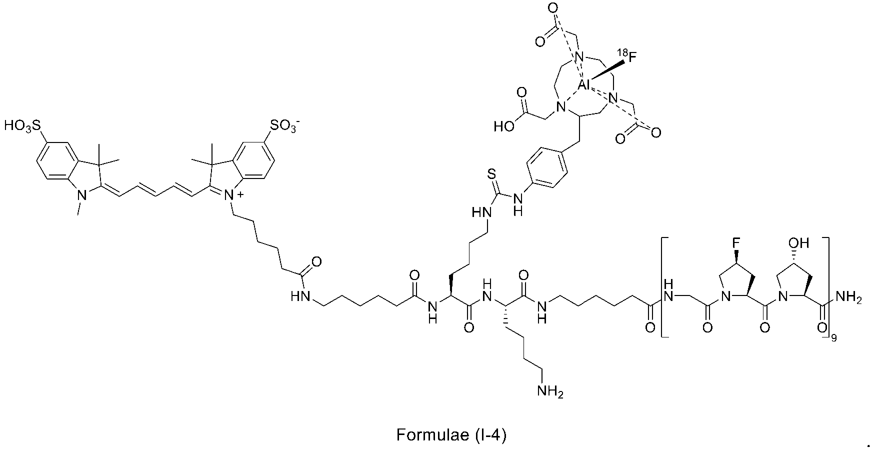

In some embodiments of the present disclosure, when the linker is 6-aminohexanoic acid, a chemical structural formula of the radiolabeled CHP is as shown in Formulae (II): 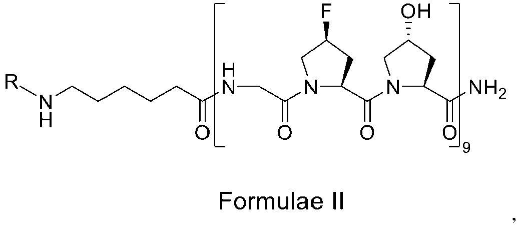 wherein, R is any one selected from the group consisting of Formula (a) to Formula (c): 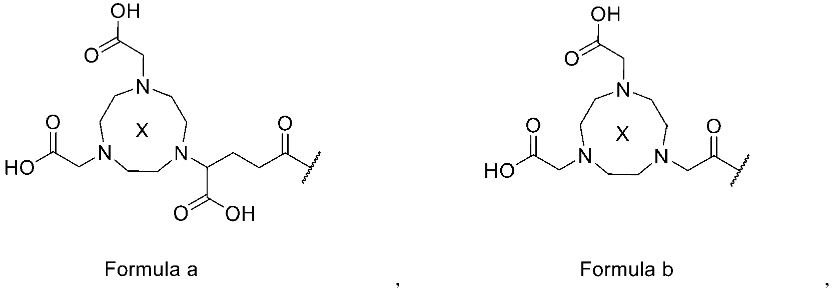 and 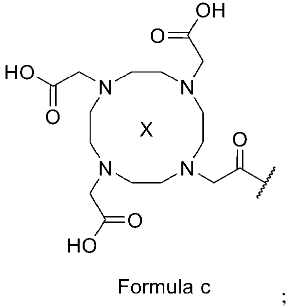 wherein, X is a radionuclide which is any one selected from the group consisting of Al¹⁸F, ⁶⁴Cu, ⁶⁷Cu, ⁶⁷Ga, ⁶⁸Ga, ⁹⁹mTc, ⁸⁹Zr, ¹¹¹In, ¹⁷⁷Lu, ¹⁸⁶Re, and ²²⁵Ac.

In some embodiments of the present disclosure, when the linker is 6-aminohexanoic acid, the radiolabeled CHP is a compound represented by any one of Formulae (II-1) to Formulae (II-4): 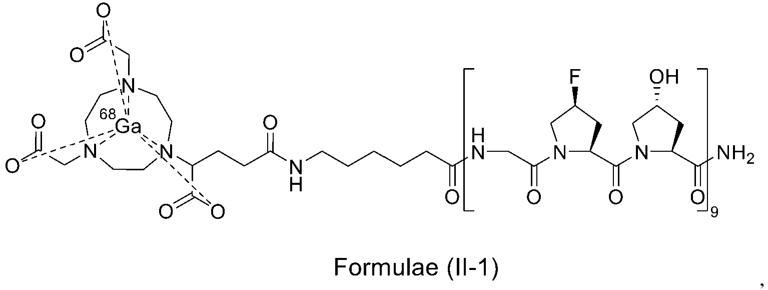 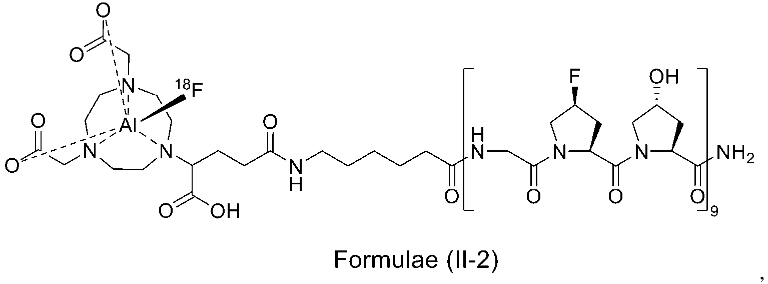 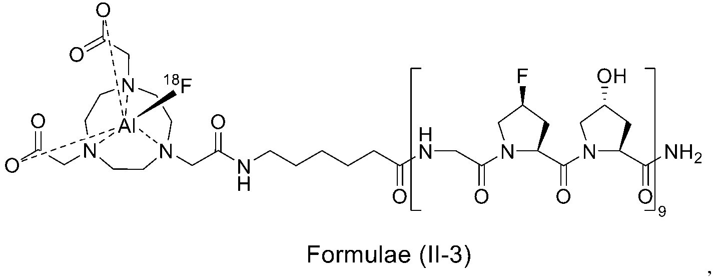 and

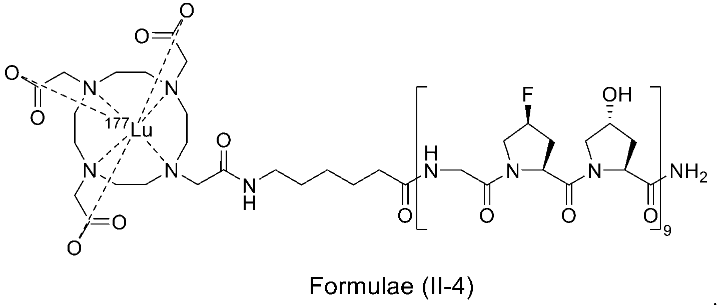

**In** the second aspect, the present disclosure provides a method for preparing the radiolabeled CHP according to the first aspect of the present disclosure, comprising the following steps:
obtaining a precursor probe comprising the chelator and the CHP by a solid-phase peptide synthesis method, and subsequently labeling the precursor probe with the radionuclide to obtain the radiolabeled CHP.

The preparation method of the radiolabeled CHP of the present disclosure is simple, and the prepared CHP exhibits good stability and a high radiochemical yield.

**In** some embodiments of the present disclosure, the method further comprises deprotection group treatment after obtaining the precursor probe.

**In** some embodiments of the present disclosure, when the linker of the radiolabeled CHP is 6-aminohexanoic acid, the preparation method comprises the following steps:
S1. reacting NH₂-Linker-CHP with NODAGA-tris(t-Bu ester), NOTA-bis(t-Bu ester), or DOTA-tris(t-Bu ester) to obtain NODAGA-Linker-CHP, NOTA2-Linker-CHP, or DOTA-Linker-CHP, wherein the linker is 6-aminohexanoic acid, and the CHP is a collagen hybridizing peptide having an amino acid sequence of (GfO)ₙ or (GPO)ₙ, where n is any positive integer between 6 and 12; and
S2. labeling the NODAGA-Linker-CHP, NOTA2-Linker-CHP, or DOTA-Linker-CHP with the radionuclide, followed by purification to obtain the radiolabeled CHP.

In some embodiments of the present disclosure, the NOTA2 has a structure as shown in Formula (a-1):

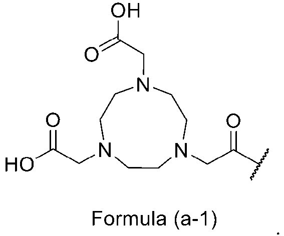

In some embodiments of the present disclosure, the NH₂-Linker-CHP is prepared by an Fmoc chemical solid-phase peptide synthesis method.

In some embodiments of the present disclosure, when the radionuclide is ⁶⁸Ga, a method for labeling comprises: reacting the NODAGA-Linker-CHP, NOTA-Linker-CHP, or DOTA-Linker-CHP with ⁶⁸GaCl₃ in a solution, followed by purification to obtain a labeled product.

In some embodiments of the present disclosure, when the metal nuclide is Al¹⁸F, a method for labeling comprises: reacting the NODAGA-Linker-CHP or NOTA-Linker-CHP with ¹⁸F⁻ and AlCl₃ in a solution, followed by purification to obtain a labeled product.

In some embodiments of the present disclosure, the solution comprises a sodium acetate solution.

In some embodiments of the present disclosure, the CHP is a collagen hybridizing peptide having an amino acid sequence of (GfO)₉.

In the third aspect, the present disclosure provides a conjugate, comprising the radiolabeled CHP according to the first aspect of the present disclosure, and a coupling moiety;
wherein, the coupling moiety comprises at least one selected from the group consisting of a protein, a drug, or a detectable marker.

In some embodiments of the present disclosure, the protein comprises a protein having an effect of improving biodistribution, such as albumin.

In some embodiments of the present disclosure, the drug comprises a compound having therapeutic efficacy.

In some embodiments of the present disclosure, the detectable marker comprises at least one of biotin, a spin marker, an enzyme, gold nanoparticles, and magnetic nanoparticles.

In some embodiments of the present disclosure, the spin marker includes, but is not limited to, deuterium.

In some embodiments of the present disclosure, the enzyme includes, but is not limited to, peroxidase, alkaline phosphatase, horseradish peroxidase, or acetylcholinesterase.

In the fourth aspect, the present disclosure provides an imaging agent, comprising the radiolabeled CHP probe according to any one of the first aspect of the present disclosure.

In some embodiments of the present disclosure, the imaging agent is used in positron emission tomography-computed tomography (PET-CT).

In the fifth aspect, the present disclosure provides use of the radiolabeled CHP according to the first aspect of the present disclosure in any one of the following:
A) preparation of a product for specifically targeting a structurally denatured collagen;
B) preparation of a product for diagnosis or lesion imaging of cancer characterized by fibrotic lesions;
C) preparation of a product for detecting an organ fibrotic disease;
D) evaluation or screening of an anti-pancreatic ductal adenocarcinoma drug or an anti-fibrotic drug.

In some embodiments of the present disclosure, the structurally denatured collagen is a structurally denatured collagen in extracellular matrix (ECM).

Unlike the increase of ECM collagen, which can only reflect the static fact of ECM deposition, the production of structurally denatured collagen is related to both collagen synthesis and collagen degradation; therefore, structurally denatured collagen can also reflect the dynamic changes of ECM remodeling during the progression of a disease, and the detection of structurally denatured collagen can assist in research on disease progression and drug development.

In some embodiments of the present disclosure, the use of the product for specifically targeting the structurally denatured collagen includes, but is not limited to, pancreatic cancer detection or early diagnosis, fibrotic disease detection, and inflammation or cardiovascular disease detection.

In some embodiments of the present disclosure, the pancreatic cancer detection or early diagnosis includes, but is not limited to, PDAC detection or early diagnosis, mucinous cystadenocarcinoma detection or early diagnosis, serous cystadenocarcinoma detection or early diagnosis, and the like.

In some embodiments of the present disclosure, the pancreatic cancer detection or early diagnosis includes detection of early-stage PanIN lesions.

In some embodiments of the present disclosure, the fibrotic disease includes, but is not limited to, pulmonary fibrosis, alcoholic liver disease, liver fibrosis, and renal fibrosis.

Pulmonary fibrosis is a group of diseases with diverse etiologies, including idiopathic pulmonary fibrosis (IPF), which is a progressive and fatal disease where patients do not exhibit symptoms until the late stage of the disease, and no treatment currently prevents or reverses its progression, leaving patients to suffer from persistent dyspnea and decline in lung function until death. Although high-resolution computed tomography (HRCT) scans can assist in diagnosing IPF, most suspected patients still require a hazardous lung biopsy to confirm the disease. Therefore, there is a need to develop more specific diagnostic tools for IPF to guide treatment decisions. For example, the ability to non-invasively identify fibrosis through innovative molecular imaging techniques can significantly improve the treatment of patients with fibrotic lung disease. As IPF is characterized by significant ECM deposition in the lungs, the probe for specifically targeting pathological ECM of the present disclosure can also be used for the precise detection and treatment of IPF.

In some embodiments of the present disclosure, the cancer characterized by fibrotic lesions includes, but is not limited to, pancreatic cancer, breast cancer, and colorectal cancer.

In some embodiments of the present disclosure, the organ fibrotic disease includes at least one of pulmonary fibrosis, pancreatic fibrosis, liver fibrosis, cardiac fibrosis, renal fibrosis, and arterial hyperplasia.

In some embodiments of the present disclosure, the product includes a reagent, a kit, or an instrumental equipment.

In the sixth aspect, the present disclosure provides a kit for imaging tumors or organ fibrosis, comprising the radiolabeled CHP as described in the first aspect.

In some embodiments of the present disclosure, the tumors include, but are not limited to, pancreatic tumors, breast tumors, prostate tumors, gastrointestinal tumors, lung tumors, and liver tumors. Specifically, the pancreatic tumors include, but are not limited to, pancreatic ductal adenoma, pancreatic endocrine tumors, mucinous cystadenoma, serous cystadenoma, pancreatic angiolipoma, and pancreatic lymphoepithelial cysts, and the like.

In some embodiments of the present disclosure, the organ fibrosis comprises collagen lesion remodeling associated with organ fibrosis.

In some embodiments of the present disclosure, the organ fibrosis includes, but is not limited to, pulmonary fibrosis, cardiac fibrosis, liver fibrosis, renal fibrosis, and the like.

In the seventh aspect, the present disclosure provides a method for treating or diagnosing a disease associated with organ fibrosis, comprising administering to a subject the radiolabeled CHP according to any one of the first aspect of the present disclosure, the conjugate according to the third aspect of the present disclosure, or the imaging agent according to the fourth aspect of the present disclosure.

In some embodiments of the present disclosure, the disease associated with organ fibrosis includes, but is not limited to, pancreatic tumors, breast tumors, prostate tumors, gastrointestinal tumors, lung tumors, and liver tumors, as well as pulmonary fibrosis, cardiac fibrosis, liver fibrosis, renal fibrosis, and the like.

In some embodiments of the present disclosure, the radiolabeled CHP, acting as a molecular probe, may be administered to a subject through, for example, systemic, local, and/or parenteral administration methods. These administration methods include, for example, injection, infusion, deposition, implantation, or topical administration, or any other administration method desired to deliver the molecular probe into tissues. In one example, the administration of the molecular probe may be performed via intravenous injection to the subject. The probe may be administered in a single dose or multiple doses.

In some embodiments of the present disclosure, the radiolabeled CHP, acting as a molecular probe, may be administered to a subject in a detectable amount in a form of a pharmaceutical composition comprising the molecular probe or a pharmaceutically acceptable water-soluble salt thereof suitable for the subject.

In some embodiments of the present disclosure, a formulation of the radiolabeled CHP to be administered will vary according to the selected route of administration, such as solution, emulsion, capsule, etc. Suitable pharmaceutically acceptable carriers may contain inert ingredients that do not unduly inhibit the biological activity of the compound. The pharmaceutically acceptable carrier should be biocompatible, for example, non-toxic, non-inflammatory, non-immunogenic, and should not cause other undesired reactions upon administration to a subject. Standard pharmaceutical formulation techniques may be employed, such as those described in Remington's Pharmaceutical Sciences, supra. Pharmaceutical carriers suitable for parenteral administration include, for example, sterile water, physiological saline, bacteriostatic saline (saline containing approximately 0.9% mg/mL benzyl alcohol), phosphate-buffered saline (PBS), Hank's solution, Ringer's lactate solution, and the like.

The beneficial effects of the present disclosure include:
(1) The present disclosure prepares a radionuclide-labeled molecular probe capable of detecting collagen structural denaturation in the ECM by coupling a radionuclide with a collagen hybridizing peptide (CHP). This radionuclide-labeled molecular probe exhibits excellent clarity and a high signal-to-noise ratio, which is capable of not only detecting PDAC in mouse pancreatic cancer models but also sensitively detecting early-stage PanIN lesions, as well as pulmonary fibrosis foci in mouse bleomycin-induced pulmonary fibrosis models. This is of significant importance for achieving early diagnosis of PDAC and reflecting the dynamic changes of ECM remodeling. Furthermore, it is noteworthy that while ECM remodeling and ECM deposition are common features of fibrosis, inflammation, cardiovascular diseases, and various solid tumors, conventional collagen-targeting probes (such as CBP8-PET) typically only highlight changes in collagen content when detecting related diseases (e.g., pulmonary fibrosis). That is, they can only reflect the static fact of ECM deposition based on the increase in ECM collagen content. In contrast, the radionuclide-labeled molecular probe of the present disclosure targets structurally denatured collagen. Since the production of structurally denatured collagen is associated with both ECM collagen synthesis and ECM collagen degradation, the probe of the present disclosure can reflect not only the static fact of ECM deposition but also the dynamic changes of ECM remodeling during disease progression, thereby possessing broader application value compared to conventional collagen-targeting molecular probes.
(2) The radiolabeled CHP probe of the present disclosure possesses characteristics of radiochemical labeling stability and exhibits excellent biodistribution profiles, characterized by high uptake in target organs such as tumors and low uptake in non-target organs such as the liver and/or kidneys, demonstrating promising clinical application prospects.

In the present disclosure:
The terms "comprise" "comprising" "include" "including" "has/have" and "having" are used in an all-inclusive, open-ended sense, meaning that other elements may be included. As used herein, the terms "such as" and "for example" are non-limiting and are for illustrative purposes only. The terms "including" and "including but not limited to" are used interchangeably.

The term "reagent" as used herein refers to a chemical compound, a mixture of chemical compounds, a biological macromolecule, or an extract prepared from biological materials.

The terms "peptide(s)," "protein(s)," and "polypeptide(s)" are used interchangeably herein. As used herein, "polypeptide" refers to any peptide or protein comprising two or more amino acids, wherein the two or more amino acids are connected to each other by peptide bonds or modified peptide bonds (i.e., peptide isosteres). "Polypeptides" refers to both short chains (often referred to as peptides, oligopeptides, or oligomers) and longer chains (often referred to as proteins).

The term "treatment" refers to both therapeutic treatment and prophylactic or preventative measures, wherein the object is to prevent or slow down (lessen) an undesired physiological change or disorder, such as cancer growth, development, or spread. For example, beneficial or desired clinical results include, but are not limited to, alleviation of symptoms, diminishment of the extent of disease, stabilized (i.e., non-worsening) state of disease, delay or slowing of disease progression, amelioration and remission of the disease state (whether partial or total), whether detectable or undetectable. "Treatment" can also refer to prolonging survival as compared to expected survival if not receiving treatment. Subjects in need of treatment include those already with the condition or disorder, those prone to have or suspected to have the condition or disorder, or those in whom the condition or disorder is to be prevented.

The term "administration" refers to providing or delivering the radiolabeled CHP in an amount and for a period of time effective to label cancer cells or fibrotic tissue in a subject.

Other features and advantages of the present disclosure will be set forth in the subsequent description.

### BRIEF DESCRIPTION OF DRAWINGS

The present disclosure will be further described below in conjunction with the accompanying drawings and embodiments, wherein:
FIG. 1A and FIG. 1B are schematic diagrams of the synthesis reaction and the chemical structure of the structurally denatured collagen-targeting probe ⁶⁸Ga -Cym-CHP of the present disclosure, wherein FIG. 1A shows the synthesis reaction formula of the ⁶⁸Ga -Cym-CHP probe, and FIG. 1B shows the chemical structural formula of the ⁶⁸Ga -Cym-CHP probe.
FIG. 2 is a schematic diagram of collagen unwinding denaturation and probe labeling within tissues during the occurrence and development of tumors and fibrosis.
FIG. 3 shows the radioactive thin layer chromatography (TLC) detection results of a free ⁶⁸GaCl₃ solution before radiolabeling and ⁶⁸Ga-Cy3-CHP after radiolabeling.
FIG. 4A and FIG. 4B show the radioactive TLC detection results of the ⁶⁸Ga-Cy3-CHP probe after incubation in different solutions for 0-2 h, wherein FIG. 4A shows the result in 1 ×PBS solution and FIG. 4B shows the result in 10% FBS solution.
FIG. 5A to FIG. 5D show the results of in vivo detection of subcutaneous PDAC tumors using the ⁶⁸Ga-Cy3-CHP probe, wherein FIG. 5A shows representative PET images obtained at 30 min and 60 min during 1 h dynamic PET imaging after injecting ⁶⁸Ga-Cy3-CHP, ⁶⁸Ga-Cy3-^{S}CHP, or performing blocking in subcutaneous tumor-bearing mice; FIG. 5B and FIG. 5C are statistical results of tumor radioactivity quantification, respectively; and FIG. 5D shows the results of ex vivo quantitative analysis of tumor uptake after 1 h dynamic scanning.
FIG. 6 is a schematic diagram of an orthotopic tumor model.
FIG. 7A and FIG. 7B show a 2 h static PET scan image and ex vivo PET results of the pancreas after tail vein injection of the nuclide probe ⁶⁸Ga-Cy5-CHP into the orthotopic tumor model, wherein FIG. 7A is the static PET scan image, and FIG. 7B shows the ex vivo PET results of the pancreas.
FIG. 8 is a statistical graph showing the probe uptake in the pancreatic tail (tumor) and the ratio of the pancreatic tail (tumor) to the pancreatic head, measured after the mice were sacrificed following imaging.
FIG. 9 is a schematic diagram of the KPC mouse model.
FIG. 10 shows a 2 h static PET scan image of the ⁶⁸Ga-Cy5-CHP probe targeting KPC lesions.
FIG. 11A and FIG. 11B show the ex vivo PET results of the pancreas and the biodistribution results after the ⁶⁸Ga-Cy5-CHP probe targeted the KPC lesions, wherein FIG. 11A is the ex vivo PET results of the pancreas, and FIG. 11B is the biodistribution results.
FIG. 12 shows 2 h and 8 h static PET scan images and ex vivo PET results of the pancreas for ⁶⁴Cu-Cy5-CHP.
FIG. 13A to FIG. 13D show the PDAC detection results of the Al¹⁸F-Cy5-CHP in vivo imaging in the KPC model, wherein FIG. 13A is a 3 h static PET scan image of Al¹⁸F-Cy5-CHP; FIG. 13B is a 1 h static PET scan image of ¹⁸F-FDG in the same mouse; FIG. 13C is the ex vivo PET result of the pancreas 3 h after injection of Al¹⁸F-Cy5-CHP; and FIG. 13D shows the biodistribution results.
FIG. 14A to FIG. 14C show the in vivo imaging results for detection of PanIN lesions using Al¹⁸F-Cy5-CHP, wherein FIG. 14A is a representative PET image of a 2 h static scan of Al¹⁸F-Cy5-CHP; FIG. 14B is the ex vivo PET result of the pancreas; and FIG. 14C is the quantification of pancreatic radioactivity uptake.
FIG. 15 shows the HE staining and CHP and CK19 fluorescence staining results of pancreatic frozen sections from low-week-old KPC mice.

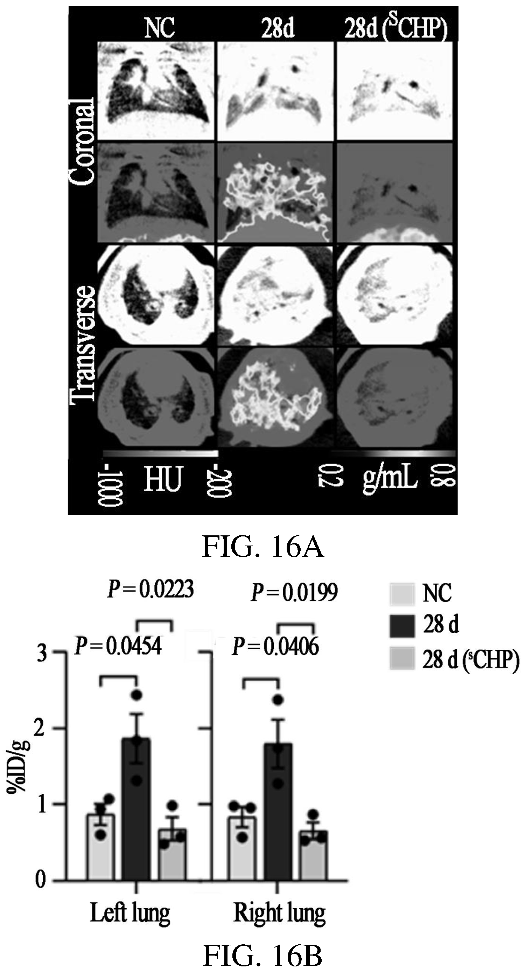

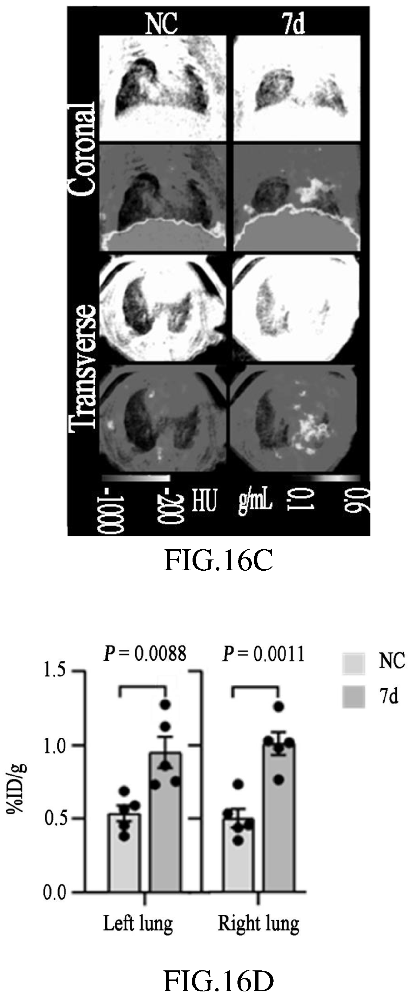

FIG. 16A to FIG. 16D show the results of detection and staging of collagen remodeling by using ⁶⁸Ga-Cy5-CHP during the progression of pulmonary fibrosis in mice, wherein FIG. 16A shows representative PET images of normal mice and pulmonary fibrosis-stage mice 28 days after bleomycin administration, injected with ⁶⁸Ga-Cy5-CHP or ⁶⁸Ga-Cy5-^{S}CHP; FIG. 16B is the statistical result of lung uptake quantification; FIG. 16C shows representative PET images of normal mice and inflammatory-stage mice 7 days after bleomycin administration, injected with ⁶⁸Ga-Cy5-CHP; and FIG. 16D is the statistical result of lung uptake quantification.
FIG. 17A to FIG. 17D show CHP hybridization revealing the response of mice to anti-fibrotic therapy, wherein FIG. 17A is the administration schedule of pirfenidone (PFD); FIG. 17B shows representative ex vivo fluorescence images of lungs from mice in the Normal, BM, and PFD groups, indicating a reduction in fluorescence signal in the PFD-treated lungs relative to untreated BM-injured lungs; FIG. 17C is the administration schedule of dihydrexidine (DHX); and FIG. 17D shows representative ex vivo fluorescence images of lungs from mice in the Normal, BM, and DHX groups.
FIG. 18 shows radioactive HPLC chromatograms of the probe precursor and the radiolabeled probe during the preparation of the probe ⁶⁸Ga-NODAGA-Ahx-(GfO)₉ in Example 5 of the present disclosure.
FIG. 19 shows radioactive HPLC chromatograms of the probe precursor and the radiolabeled probe during the preparation of the probe Al¹⁸F-NODAGA-Ahx-(GfO)₉ in Example 6 of the present disclosure.
FIG. 20 shows radioactive HPLC chromatograms of the probe precursor and the radiolabeled probe during the preparation of the probe Al¹⁸F-NOTA2-Ahx-(GfO)₉ in Example 7 of the present disclosure.
FIG. 21 shows radioactive HPLC chromatograms of the probe precursor and the radiolabeled probe during the preparation of the probe Cy5-Ahx-K(NOTA-Al¹⁸F)-K(NH₂)-Ahx-(GfO)₉ in Comparative Example 4 of the present disclosure.
FIG. 22 shows the Matrix-Assisted Laser Desorption/Ionization (MALDI) spectrum of the probe in Comparative Example 4 of the present disclosure.
FIG. 23 shows radioactive HPLC chromatograms of the probe precursor and the radiolabeled probe during the preparation of the probe Al¹⁸F-NOTA-Ahx-(GfO)₉ in Comparative Example 6 of the present disclosure.
FIG. 24 shows the 1 h PET/CT results of the probe prepared in Example 5 of the present disclosure in pancreatic cancer subcutaneous tumor-bearing mice.
FIG. 25 shows the 1 h PET/CT results of the probe prepared in Example 7 of the present disclosure in pancreatic cancer subcutaneous tumor-bearing mice and normal mice.
FIG. 26 shows the 1 h PET/CT results of the probe prepared in Comparative Example 3 of the present disclosure in pancreatic cancer subcutaneous tumor-bearing mice.
FIG. 27 shows the 1 h PET/CT results of the probe prepared in Comparative Example 4 of the present disclosure in normal mice.
FIG. 28 shows the 1 h PET/CT results of the probe prepared in Comparative Example 5 of the present disclosure in pancreatic cancer subcutaneous tumor-bearing mice.
FIG. 29 shows the 1 h PET/CT results of the probe prepared in Comparative Example 6 of the present disclosure in pancreatic cancer subcutaneous tumor-bearing mice.

### DETAILED DESCRIPTION

The concepts and technical effects of the present disclosure will be described clearly and completely in conjunction with the embodiments below to fully understand the objectives, features, and effects of the present disclosure. Obviously, the described embodiments are only a part of the embodiments of the present disclosure, rather than all of them. Other embodiments obtained by those skilled in the art based on the embodiments of the present disclosure without creative labor shall fall within the scope of protection of the present disclosure.

The words "preferably," "more preferably," and the like in the present disclosure refer to embodiments of the present disclosure that may provide certain beneficial effects under certain circumstances. However, under the same or other circumstances, other embodiments may also be preferred. Furthermore, the expression of one or more preferred embodiments does not imply that other embodiments are unavailable, nor is it intended to exclude other embodiments from the scope of the present disclosure.

When a numerical range is disclosed herein, the range is regarded as continuous and includes the minimum and maximum values of the range, as well as every value between such minimum and maximum values. Further, when the range refers to integers, every integer between the minimum and maximum values of the range is included. In addition, when multiple ranges are provided to describe a feature or characteristic, the ranges may be combined. In other words, unless otherwise specified, all ranges disclosed herein are understood to include any and all sub-ranges subsumed therein.

In the description of the present disclosure, descriptions referencing the terms "one embodiment," "some embodiments," "an illustrative embodiment," "an example," "a specific example," or "some examples," and the like mean that specific features, structures, materials, or characteristics described in connection with the embodiment or example are included in at least one embodiment or example of the present disclosure. In this specification, the schematic expressions of the above terms do not necessarily refer to the same embodiment or example. Moreover, the specific features, structures, materials, or characteristics described may be combined in any one or more embodiments or examples in an appropriate manner.

In the embodiments of the present disclosure, GfO is a glycine-fluorinated proline-hydroxyproline triplet, and the n units of (GfO) together constitute a collagen hybridizing peptide (CHP). The CHP possesses a propensity for triple-helix formation and can hybridize with incomplete or unfolded collagen peptide chains after degradation to form a triple helix, thereby labeling damaged and denatured collagen in pathologically injured tissues, while not interacting with intact structured collagen or other proteins lacking a triple-helix structure.

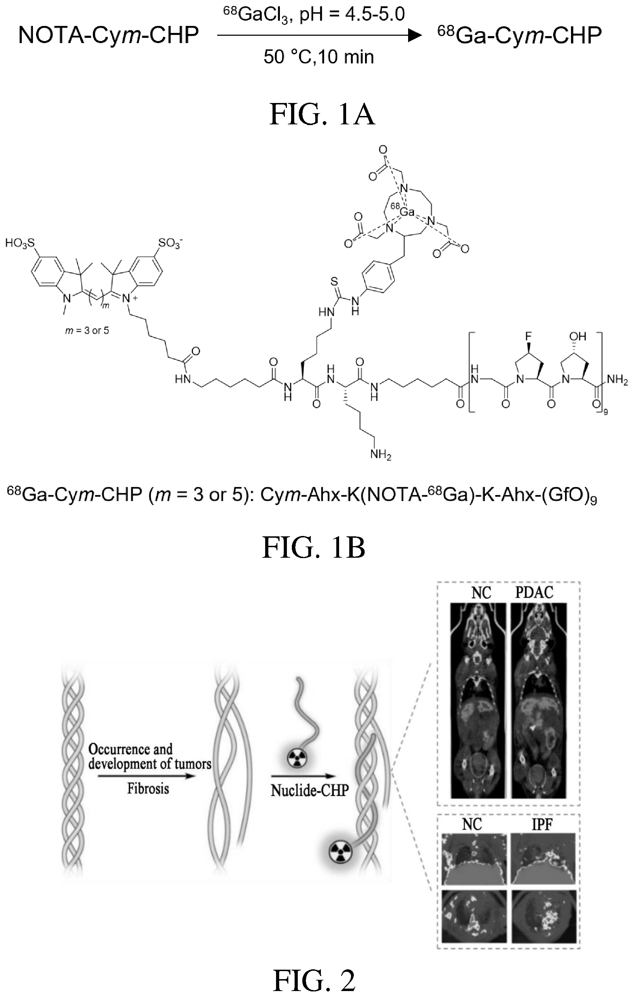

In the embodiments of the present disclosure, the schematic diagrams of the synthesis of ⁶⁸Ga-Cym-CHP probe are shown in FIG. 1A and FIG. 1B, wherein FIG. 1A shows the synthesis reaction formula of ⁶⁸Ga-Cym-CHP, and FIG. 1B shows the chemical structural formula of the structurally denatured collagen-targeting probe ⁶⁸Ga-Cym-CHP. The schematic diagram of the binding and labeling of the nuclide-labeled CHP probe to the unwound and denatured collagen within the tissue is shown in FIG. 2.

In the embodiments of the present disclosure, ⁶⁸Ga may be replaced by other radioactive elements, such as ⁶⁴Cu or ¹⁸F, and the like; the NOTA chelator may be replaced by other metal chelators, such as NODAGA; and (GfO)ₙ may be replaced by (GPO)ₙ having the same propensity for triple-helix formation.

The present disclosure describes methods involving conventional molecular biology techniques. Such techniques are well known in the art and are described in detail in methodological treatises, for example, Current Protocols in Molecular Biology, ed. Ausubel et al., Greene Publishing and Wiley-Interscience, New York, 1992. Unless otherwise defined, all technical terms used herein have the same meaning as commonly understood by one of ordinary skill in the art to which this application belongs. Commonly understood definitions of molecular biology terms can be found in, for example, Rieger et al., Glossary of Genetics: Classical and Molecular, 5th Edition, Springer-Verlag: New York, 1991, and Lewin, Genes V, Oxford University Press: New York, 1994.

Where specific conditions are not indicated in the embodiments, they are carried out according to conventional conditions or conditions recommended by the manufacturer. Where the manufacturer of the reagents or instruments used is not indicated, they are all conventional products that are commercially available.

### Example 1: Synthesis of ⁶⁸Ga-Cy3-CHP

This example describes the design of a radiolabeled CHP probe that can specifically target unwound collagen in pathological ECM. The sequence of the probe is Cy3-Ahx-K(NOTA-⁶⁸Ga)-K(NH₂)-Ahx-(GfO)₉, abbreviated as ⁶⁸Ga-Cy3-CHP, wherein Ahx is 6-aminohexanoic acid, K is lysine (Lys), Cy3 is the cyanine dye sulfo-Cy3, NOTA is the chelator p-SCN-Bn-NOTA used for chelating the radioactive metal nuclide ⁶⁸Ga, and GfO is a glycine-fluorinated proline-hydroxyproline triplet, with nine (GfO) units collectively constituting the collagen hybridizing peptide.

The structural formula of the ⁶⁸Ga-Cy3-CHP probe in this example is as shown in Formula (I-1):

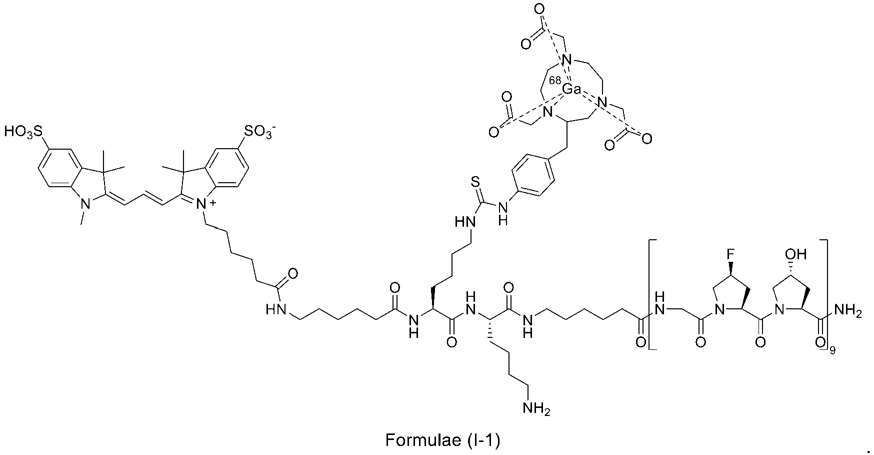

The synthesis of the probe specifically included the following steps:

### (1) Synthesis of the precursor probe NOTA-Cy3-CHP

First, using a PurePep Chorus peptide synthesizer, Ahx-K(Boc)-K(Dde)-Ahx-(GfO)₉-Rink Amide AM resin was obtained on Rink Amide AM resin by employing standard Fmoc solid-phase peptide synthesis. The Fmoc amino acids included Fmoc-Gly-OH, Fmoc-Pro-OH, Fmoc-Hyp-(tBu)-OH, Fmoc-Ahx-OH, Fmoc-Lys(Dde)-OH, and Fmoc-Lys(Boc)-OH.

The sulfonated Cy3 cyanine dye was coupled to the N-terminal amino group of the peptide on the resin via Fmoc solid-phase peptide synthesis. The peptide Cy3-Ahx-K(NH₂)-K(Dde)-Ahx-(GfO)₉ was cleaved from the resin, purified by high-performance liquid chromatography (HPLC), and then lyophilized. After the lyophilized peptide reacted with p-SCN-Bn-NOTA for 24 h, the Dde-protecting group of lysine was removed using 3% hydrazine hydrate. The obtained product was then purified by HPLC and lyophilized to obtain the precursor probe Cy3-Ahx-K(NOTA)-K(NH₂)-Ahx-(GfO)₉, hereinafter referred to as NOTA-Cy3-CHP.

### (2) ⁶⁸Ga Radiolabeling

24 nmol of NOTA-Cy3-CHP was dissolved in approximately 600 µL of 0.5 M sodium acetate buffer (pH 5.5), and then reacted with 222 MBq of ⁶⁸GaCl₃ at 50 °C for 10 minutes. Subsequently, the reaction mixture was filtrated with a 0.22 µm Millipore filter to obtain the final product ⁶⁸Ga-Cy3-CHP.

### Example 2: Synthesis of ⁶⁸Ga-Cy5-CHP

This example describes the design of another radiolabeled CHP probe that can specifically target unwound collagen in pathological ECM. The sequence of the probe is Cy5-Ahx-K(NOTA-⁶⁸Ga)-K(NH₂)-Ahx-(GfO)₉, abbreviated as ⁶⁸Ga-Cy5-CHP, wherein Ahx is 6-aminohexanoic acid, K is lysine (Lys), Cy5 is a cyanine dye, and NOTA is the chelator p-SCN-Bn-NOTA used for chelating the radioactive metal nuclide ⁶⁸Ga.

The structural formula of the ⁶⁸Ga-Cy5-CHP probe in this example is as shown in Formula (I-2):

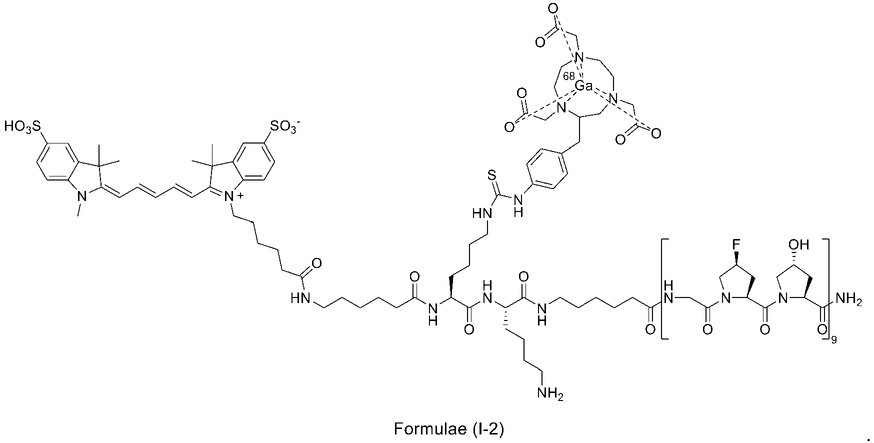

The synthesis of this probe is similar to that of the ⁶⁸Ga-Cy3-CHP probe in Example 1 above, specifically including the following steps:

### (1) Synthesis of the precursor probe NOTA-Cy5-CHP

Using a PurePep Chorus peptide synthesizer, Ahx-K(Boc)-K(Dde)-Ahx-(GfO)₉-Rink Amide AM resin was obtained on Rink Amide AM resin by employing standard Fmoc solid-phase peptide synthesis. The Fmoc amino acids included Fmoc-Gly-OH, Fmoc-Pro-OH, Fmoc-Hyp-(tBu)-OH, Fmoc-Ahx-OH, Fmoc-Lys(Dde)-OH, and Fmoc-Lys(Boc)-OH.

The sulfonated Cy5 cyanine dye was coupled to the N-terminal amino group of the peptide on the resin via Fmoc solid-phase peptide synthesis. The peptide Cy5-Ahx-K(NH₂)-K(Dde)-Ahx-(GfO)₉ was cleaved from the resin, purified by HPLC, and then lyophilized. After the lyophilized peptide reacted with p-SCN-Bn-NOTA for 24 h, the Dde-protecting group of lysine was removed using 3% hydrazine hydrate. The obtained product was then purified by HPLC and lyophilized to obtain the precursor probe Cy5-Ahx-K(NOTA)-K(NH₂)-Ahx-(GfO)₉, hereinafter referred to as NOTA-Cy5-CHP.

### (2) ⁶⁸Ga Radiolabeling

24 nmol of NOTA-Cy5-CHP was dissolved in approximately 600 µL of 0.5 M sodium acetate buffer (pH 5.5), and then reacted with 222 MBq of ⁶⁸GaCl₃ at 50 °C for 10 minutes. Subsequently, the reaction mixture was filtrated with a 0.22 µm Millipore filter to obtain the final product ⁶⁸Ga-Cy5-CHP.

### Example 3: Synthesis of ⁶⁴Cu-Cy5-CHP

This example improves upon ⁶⁸Ga-Cy5-CHP by using another metal nuclide, ⁶⁴Cu, to prepare Cy5-Ahx-K(NOTA-⁶⁴Cu)-K(NH₂)-Ahx-(GfO)₉, hereinafter referred to as ⁶⁴Cu-Cy5-CHP probe. Its structural formula is as shown in Formula (I-3):

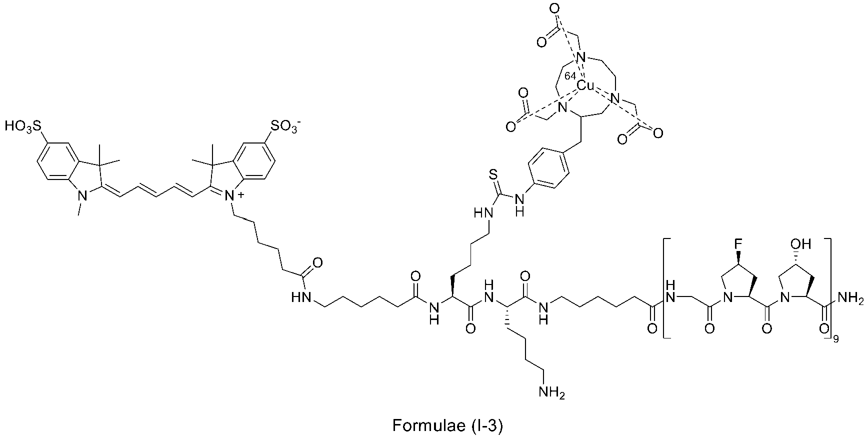

The preparation of this probe utilized the same precursor probe NOTA-Cy5-CHP as described in Example 2, followed by radiolabeling with ⁶⁴Cu, specifically comprising the following steps:

### (1) Synthesis of the precursor probe NOTA-Cy5-CHP

Using a PurePep Chorus peptide synthesizer, Ahx-K(Boc)-K(Dde)-Ahx-(GfO)₉-Rink Amide AM resin was obtained on Rink Amide AM resin by employing standard Fmoc solid-phase peptide synthesis. The Fmoc amino acids included Fmoc-Gly-OH, Fmoc-Pro-OH, Fmoc-Hyp-(tBu)-OH, Fmoc-Ahx-OH, Fmoc-Lys(Dde)-OH, and Fmoc-Lys(Boc)-OH.

The sulfonated Cy5 cyanine dye was coupled to the N-terminal amino group of the peptide on the resin via Fmoc solid-phase peptide synthesis. The peptide Cy5-Ahx-K(NH₂)-K(Dde)-Ahx-(GfO)₉ was cleaved from the resin, purified by HPLC, and then lyophilized. After the lyophilized peptide reacted with p-SCN-Bn-NOTA for 24 h, the Dde-protecting group of lysine was removed using 3% hydrazine hydrate. The obtained product was then purified by HPLC and lyophilized to obtain the precursor probe Cy5-Ahx-K(NOTA)-K(NH₂)-Ahx-(GfO)₉, hereinafter referred to as NOTA-Cy5-CHP.

### (2) ⁶⁴Cu Radiolabeling

24 nmol of NOTA-Cy5-CHP was dissolved in approximately 600 µL of 0.5 M sodium acetate buffer (pH 5.5), and then reacted with 222 MBq of ⁶⁴CuCl₂ at 50 °C for 10 minutes. Subsequently, the reaction mixture was filtrated with a 0.22 µm Millipore filter to obtain the final product ⁶⁴Cu-Cy5-CHP.

### Example 4: Synthesis of Al¹⁸F-Cy5-CHP

This example provides another radiolabeled CHP probe, Cy5-Ahx-K(NOTA-Al¹⁸F)-K(NH₂)-Ahx-(GfO)₉, hereinafter referred to as Al¹⁸F-Cy5-CHP, which can specifically target unwound collagen in pathological ECM. The difference from Example 2 lies in changing the nuclide to Al¹⁸F. The structural formula of Al¹⁸F-Cy5-CHP probe is as shown in Formula (I-4):

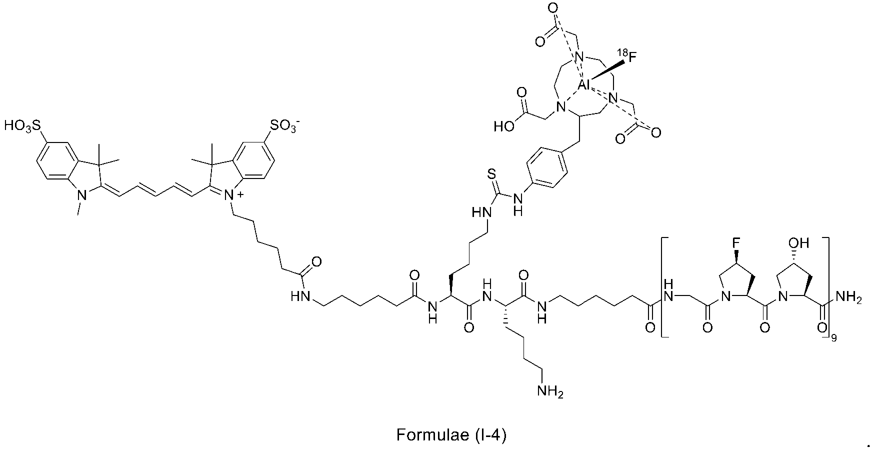

The specific preparation process of the probe was as follows:

### (1) Synthesis of the precursor probe NOTA-Cy5-CHP

Using a PurePep Chorus peptide synthesizer, Ahx-K(Boc)-K(Dde)-Ahx-(GfO)₉-Rink Amide AM resin was obtained on Rink Amide AM resin by employing standard Fmoc solid-phase peptide synthesis. The Fmoc amino acids included Fmoc-Gly-OH, Fmoc-Pro-OH, Fmoc-Hyp-(tBu)-OH, Fmoc-Ahx-OH, Fmoc-Lys(Dde)-OH, and Fmoc-Lys(Boc)-OH.

The sulfonated Cy5 cyanine dye was coupled to the N-terminal amino group of the peptide on the resin via Fmoc solid-phase peptide synthesis. The peptide Cy5-Ahx-K(NH₂)-K(Dde)-Ahx-(GfO)₉ was cleaved from the resin, purified by HPLC, and then lyophilized. After the lyophilized peptide reacted with p-SCN-Bn-NOTA for 24 h, the Dde-protecting group of lysine was removed using 3% hydrazine hydrate. The obtained product was then purified by HPLC and lyophilized to obtain the precursor probe Cy5-Ahx-K(NOTA)-K(NH₂)-Ahx-(GfO)₉, hereinafter referred to as NOTA-Cy5-CHP.

### (2) Al¹⁸F Radiolabeling

10 µL of AlCl₃ in sodium acetate buffer (pH 4.0) was added to ¹⁸F-sodium acetate buffer (100 µL, 30 mCi, pH 4.0), mixed, and left at room temperature for 5 min to form the [Al¹⁸F]²⁺ conjugate. Subsequently, 40 µL of the prepared NOTA-Cy5-CHP (1 mM, 40 nmol) was added, and the mixture was heated at 110 °C for 30 min. The reaction solution was cooled to room temperature, diluted with 3 mL of ultrapure water, and passed through a Sep-Pak C18-Light column. Then, the column was washed with 10-20 mL of ultrapure water to remove unreacted Al¹⁸F. Finally, the obtained product was eluted with anhydrous ethanol and concentrated by heating to obtain Al¹⁸F-Cy5-CHP.

### Example 5: ⁶⁸Ga-NODAGA-Ahx-(GfO)₉

This example provides a radiolabeled CHP, ⁶⁸Ga-NODAGA-Ahx-(GfO)₉, wherein NODAGA is the chelator, Ahx is 6-aminohexanoic acid, and (GfO)₉ is the collagen hybridizing peptide. The chemical structural formula of this probe is as shown in Formula (II-1):

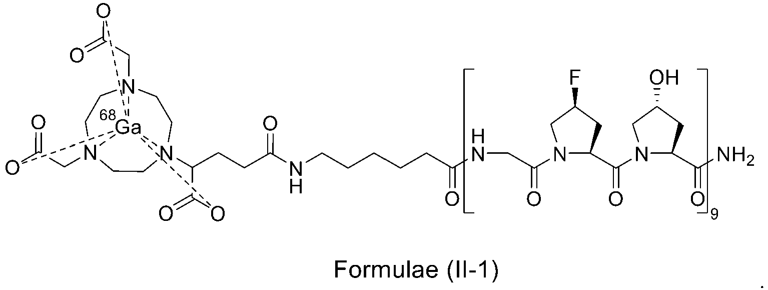

The preparation method for the above-mentioned ⁶⁸Ga-NODAGA-Ahx-(GfO)₉ was as follows:
(1) Synthesis of the probe precursor NODAGA-Ahx-(GfO)₉: NH₂-Ahx-(GfO)₉ was obtained on Rink Amide AM resin by employing standard Fmoc solid-phase peptide synthesis. After further reacting with NODAGA-tris(t-Bu ester) (CAS: 1190101-34-8) for 24 h, the peptide was cleaved from the resin, purified by HPLC, and lyophilized to obtain the probe precursor NODAGA-Ahx-(GfO)₉.
(2) ⁶⁸Ga radiolabeling: 50 nmol of the probe precursor NODAGA-Ahx-(GfO)₉ was dissolved in 300 mL of 0.5 M sodium acetate solution and then reacted with 666 MBq of ⁶⁸GaCl₃ at 50 °C for 10 minutes. The resulting reaction mixture was then purified using a Sep-Pak C18 column to obtain the ⁶⁸Ga-NODAGA-Ahx-(GfO)₉.

### Example 6: Al¹⁸F-NODAGA-Ahx-(GfO)₉

This example provides a radiolabeled CHP, Al¹⁸F-NODAGA-Ahx-(GfO)₉, wherein NODAGA is the chelator, Ahx is 6-aminohexanoic acid, and (GfO)₉ is the collagen hybridizing peptide. The chemical structural formula of this probe is as shown in Formula (II-2):

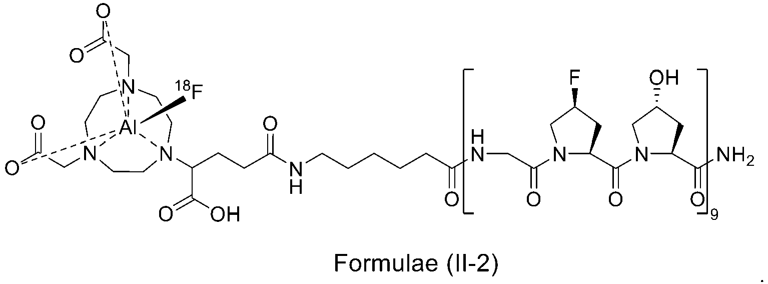

The preparation method for the above-mentioned Al¹⁸F-NODAGA-Ahx-(GfO)₉ was as follows:
(1) Synthesis of the probe precursor NODAGA-Ahx-(GfO)₉: NH₂-Ahx-(GfO)₉ was obtained on Rink Amide AM resin by employing standard Fmoc solid-phase peptide synthesis. After further reacting with NODAGA-tris(t-Bu ester) (CAS: 1190101-34-8) for 24 h, the peptide was cleaved from the resin, purified by HPLC, and lyophilized to obtain the probe precursor NODAGA-Ahx-(GfO)₉.
(2) Al¹⁸F radiolabeling: ¹⁸F⁻ (0.1 mL, 2.5 GBq) was mixed with AlCl₃ (10 µL, 2 mM) and sodium acetate buffer (0.1 mL, 0.5 M, pH 4.0) at room temperature for 10 minutes. Subsequently, 50 nmol of the probe precursor NODAGA-Ahx-(GfO)₉ was added, and the mixture was heated at 110 °C for 30 minutes. The reaction mixture was then purified using a Sep-Pak C18 column to obtain the Al¹⁸F-NODAGA-Ahx-(GfO)₉.

### Example 7: Al¹⁸F-NOTA2-Ahx-(GfO)₉

This example provides a radiolabeled CHP, Al¹⁸F-NOTA2-Ahx-(GfO)₉, wherein NOTA2 is the chelator, Ahx is 6-aminohexanoic acid, and (GfO)₉ is the collagen hybridizing peptide. The chemical structural formula of this probe is as shown in Formula (II-3):

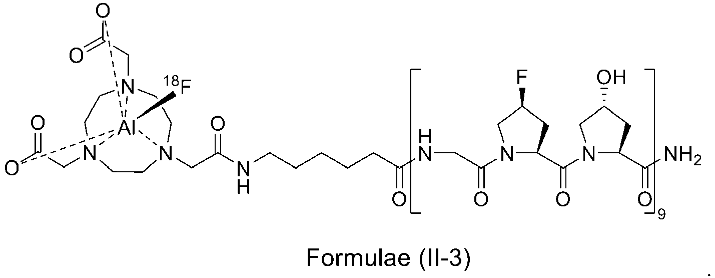

The preparation method for the above-mentioned Al¹⁸F-NOTA2-Ahx-(GfO)₉ was as follows:
(1) Synthesis of the probe precursor NOTA2-Ahx-(GfO)₉: NH₂-Ahx-(GfO)₉ was obtained on Rink Amide AM resin by employing standard Fmoc solid-phase peptide synthesis. After further reacting with NOTA-bis(t-Bu ester) (CAS: 1161415-28-6) for 24 h, the peptide was cleaved from the resin, purified by HPLC, and lyophilized to obtain the probe precursor NOTA2-Ahx-(GfO)₉.
(2) Al¹⁸F radiolabeling: ¹⁸F⁻ (0.1 mL, 2.5 GBq) was mixed with AlCl₃ (10 µL, 2 mM) and sodium acetate buffer (0.1 mL, 0.5 M, pH 4.0) at room temperature for 10 minutes. Subsequently, 50 nmol of the probe precursor NOTA2-Ahx-(GfO)₉ was added, and the mixture was heated at 110 °C for 30 minutes. The reaction mixture was then purified using a Sep-Pak C18 column to obtain the Al¹⁸F-NOTA2-Ahx-(GfO)₉.

### Comparative Example 1: Control Probe ⁶⁸Ga-Cy3-^{S}CHP

This comparative example provides a non-targeting control probe, Cy3-Ahx-K(NOTA-⁶⁸Ga)-K(NH₂)-Ahx-^{S}(GfO)₉, abbreviated as ⁶⁸Ga-Cy3-^{S}CHP. The sequence of ³(GfO)₉ is OfGGOfGfGfOfOGOfGOOfGGOOffG (SEQ ID NO: 1), wherein O is hydroxyproline, f is fluorinated proline, and G is glycine. This sequence was obtained by randomly scrambling the targeting sequence (GfO)₉.

The preparation process of the ⁶⁸Ga-Cy3-^{S}CHP probe differs from Example 1 only in the targeting sequence, while all other conditions remain the same.

### Comparative Example 2: Control Probe ⁶⁸Ga-Cy5-^{S}CHP

This comparative example provides a non-targeting control probe, Cy5-Ahx-K(NOTA-⁶⁸Ga)-K(NH₂)-Ahx-^{S}(GfO)₉, abbreviated as ⁶⁸Ga-Cy5-^{S}CHP. The sequence of ³(GfO)₉ is OfGGOfGfGfOfOGOfGOOfGGOOffG (SEQ ID NO: 1), wherein O is hydroxyproline, f is fluorinated proline, and G is glycine. This sequence was obtained by randomly scrambling the targeting sequence (GfO)₉.

The preparation process of the ⁶⁸Ga-Cy5-^{S}CHP probe differs from Example 2 only in the targeting sequence, while all other conditions remain the same.

### Comparative Example 3: Cy5-Ahx-K(NOTA-⁶⁸Ga)-K(NH₂)-Ahx-(GfO)₉

This comparative example provides a fluorescence-radiolabeled CHP, Cy5-Ahx-K(NOTA-⁶⁸Ga)-K(NH₂)-Ahx-(GfO)₉, wherein Cy5 is a fluorescent dye moiety, Ahx is 6-aminohexanoic acid, K is a lysine residue, NOTA is a chelator, and (GfO)₉ is a collagen hybridizing peptide. The chemical structural formula of the probe is as follows:

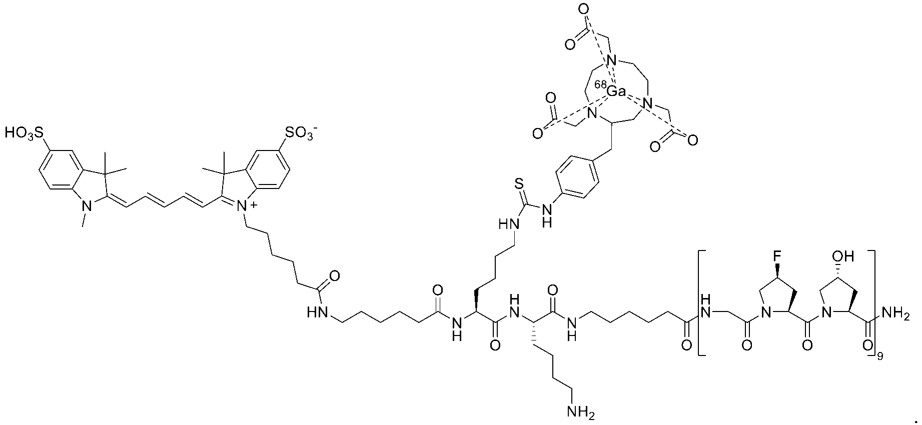

The preparation method for the above-mentioned Cy5-Ahx-K(NOTA-⁶⁸Ga)-K(NH₂)-Ahx-(GfO)₉ was as follows:
(1) Synthesis of the probe precursor Cy5-Ahx-K(NOTA)-K(NH₂)-Ahx-(GfO)₉: Using a PurePep Chorus peptide synthesizer, Ahx-K(Boc)-K(Dde)-Ahx-(GfO)₉-Rink Amide AM resin was obtained on Rink Amide AM resin by employing standard Fmoc solid-phase peptide synthesis. After the Cy5 cyanine dye was reacted onto the resin, the peptide was cleaved from the resin, purified by HPLC, and then lyophilized to obtain Cy5-Ahx-K(NH₂)-K(Dde)-Ahx-(GfO)₉. Subsequently, the obtained product was reacted with p-SCN-Bn-NOTA (CAS: 147597-66-8) for 24 h, and the Dde-protecting group of lysine was removed using hydrazine hydrate. The product was then purified by HPLC and lyophilized to obtain the probe precursor Cy5-Ahx-K(NOTA)-K(NH₂)-Ahx-(GfO)₉.
(2) ⁶⁸Ga radiolabeling: 50 nmol of the probe precursor Cy5-Ahx-K(NOTA)-K(NH₂)-Ahx-(GfO)₉ was dissolved in 300 mL of 0.5 M sodium acetate solution and then reacted with 666 MBq of ⁶⁸GaCl₃ at 50 °C for 10 minutes. The resulting reaction mixture was then purified using a Sep-Pak C18 column to obtain the Cy5-Ahx-K(NOTA-⁶⁸Ga)-K(NH₂)-Ahx-(GfO)₉.

### Comparative Example 4: Cy5-Ahx-K(NOTA-Al¹⁸F)-K(NH₂)-Ahx-(GfO)₉

This comparative example provides a fluorescence-radiolabeled CHP, Cy5-Ahx-K(NOTA-Al¹⁸F)-K(NH₂)-Ahx-(GfO)₉, wherein Cy5 is a fluorescent dye moiety, Ahx is 6-aminohexanoic acid, K is a lysine residue, NOTA is a chelator, and (GfO)₉ is a collagen hybridizing peptide. The chemical structural formula of the probe is as follows:

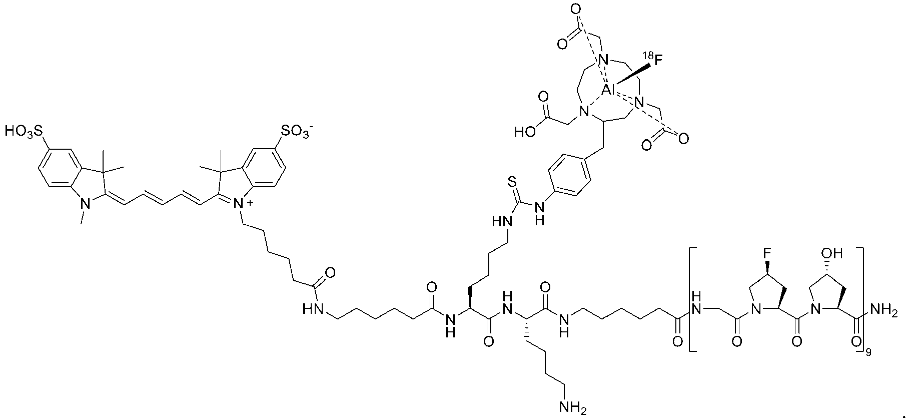

The preparation method for the above-mentioned Cy5-Ahx-K(NOTA-Al¹⁸F)-K(NH₂)-Ahx-(GfO)₉ was as follows:
(1) Synthesis of the probe precursor Cy5-Ahx-K(NOTA)-K(NH₂)-Ahx-(GfO)₉: Using a PurePep Chorus peptide synthesizer, Ahx-K(Boc)-K(Dde)-Ahx-(GfO)₉-Rink Amide AM resin was obtained on Rink Amide AM resin by employing standard Fmoc solid-phase peptide synthesis. After the Cy5 cyanine dye was reacted onto the resin, the peptide was cleaved from the resin, purified by HPLC, and then lyophilized to obtain Cy5-Ahx-K(NH₂)-K(Dde)-Ahx-(GfO)₉. Subsequently, the obtained product was reacted with p-SCN-Bn-NOTA (CAS: 147597-66-8) for 24 h, and the Dde-protecting group of lysine was removed using hydrazine hydrate. The product was then purified by HPLC and lyophilized to obtain the probe precursor Cy5-Ahx-K(NOTA)-K(NH₂)-Ahx-(GfO)₉.
(2) Al₁₈F radiolabeling: ¹⁸F⁻ (0.1 mL, 2.5 GBq) was mixed with AlCl₃ (10 µL, 2 mM) and sodium acetate buffer (0.1 mL, 0.5 M, pH 4.0) at room temperature for 10 minutes. Subsequently, 50 nmol of the probe precursor Cy5-Ahx-K(NOTA)-K(NH₂)-Ahx-(GfO)₉ was added, and the mixture was heated at 110 °C for 30 minutes. The reaction mixture was then purified using a Sep-Pak C18 column to obtain the Cy5-Ahx-K(NOTA-Al¹⁸F)-K(NH₂)-Ahx-(GfO)₉.

### Comparative Example 5: Cy5-Ahx-K(NOTA-⁶⁸Ga)-Ahx-(GfO)₉

This comparative example provides a fluorescence-radiolabeled CHP, Cy5-Ahx-K(NOTA-⁶⁸Ga)-Ahx-(GfO)₉, wherein Cy5 is a fluorescent dye moiety, Ahx is 6-aminohexanoic acid, K is a lysine residue, NOTA is a chelator, and (GfO)₉ is a collagen hybridizing peptide. The chemical structural formula of the probe is as follows:

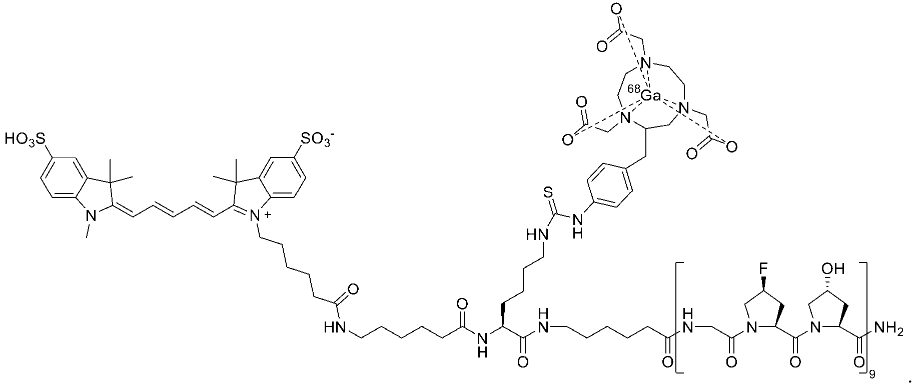

The preparation method for the above-mentioned Cy5-Ahx-K(NOTA-⁶⁸Ga)-Ahx-(GfO)₉ was as follows:
(1) Synthesis of the probe precursor Cy5-Ahx-K(NOTA)-Ahx-(GfO)₉: Cy5-Ahx-K(NH₂)-Ahx-(GfO)₉ was obtained by standard Fmoc solid-phase peptide synthesis on Rink Amide AM resin, followed by cleavage and purification via HPLC. The lyophilized peptide was reacted with p-SCN-Bn-NOTA (CAS: 147597-66-8) for 24 h, then purified by HPLC and lyophilized to obtain the probe precursor Cy5-Ahx-K(NOTA)-Ahx-(GfO)₉.
(2) ⁶⁸Ga radiolabeling: 50 nmol of the probe precursor Cy5-Ahx-K(NOTA)-Ahx-(GfO)₉ was dissolved in 300 mL of 0.5 M sodium acetate solution and then reacted with 666 MBq of ⁶⁸GaCl₃ at 50 °C for 10 minutes. The resulting reaction mixture was then purified using a Sep-Pak C18 column to obtain the Cy5-Ahx-K(NOTA-⁶⁸Ga)-Ahx-(GfO)₉.

### Comparative Example 6: Al¹⁸F-NOTA-Ahx-(GfO)₉

This comparative example provides a radiolabeled CHP, Al¹⁸F-NOTA-Ahx-(GfO)₉, wherein NOTA is a chelator, Ahx is 6-aminohexanoic acid, and (GfO)₉ is a collagen hybridizing peptide. The chemical structural formula of the probe is as follows:

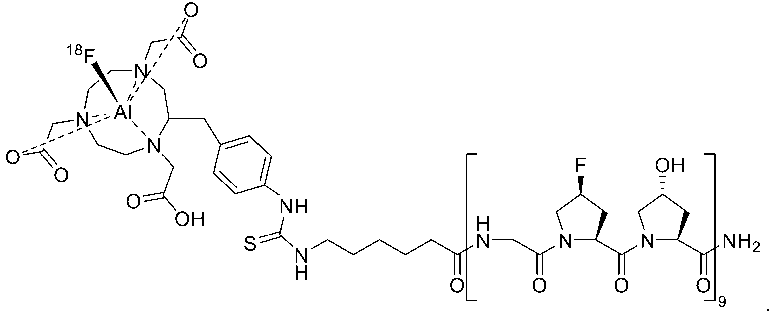

The preparation method for the above-mentioned Al¹⁸F-NOTA-Ahx-(GfO)₉ was as follows:
(1) Synthesis of the probe precursor NOTA-Ahx-(GfO)₉: NH₂-Ahx-(GfO)₉ was obtained by standard Fmoc solid-phase peptide synthesis on Rink Amide AM resin followed by cleavage and purification via HPLC. The lyophilized peptide was reacted with p-SCN-Bn-NOTA (CAS: 147597-66-8) for 24 h, then purified by HPLC and lyophilized to obtain the probe precursor NOTA-Ahx-(GfO)₉.
(2) Al¹⁸F radiolabeling: ¹⁸F⁻ (0.1 mL, 2.5 GBq) was mixed with AlCl₃ (10 µL, 2 mM) and sodium acetate buffer (0.1 mL, 0.5 M, pH 4.0) at room temperature for 10 minutes. Subsequently, 50 nmol of the probe precursor NOTA-Ahx-(GfO)₉ was added, and the mixture was heated at 110 °C for 30 minutes. The reaction mixture was then purified using a Sep-Pak C18 column to obtain the Al¹⁸F-NOTA-Ahx-(GfO)₉.

### Test Example 1: Radioactivity and In Vitro Stability Analysis

This test example used ⁶⁸Ga-Cy3-CHP prepared in Example 1 as the detection probe to analyze the radioactivity and stability of the probes prepared according to the present disclosure.

### (1) Radioactive TLC Analysis

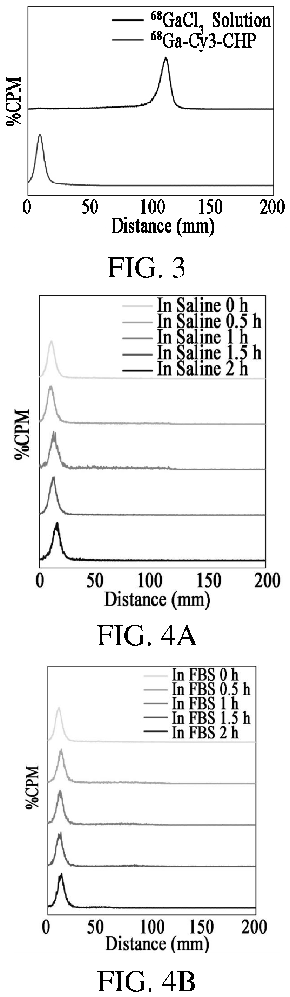

The radiochemical purity of the final product ⁶⁸Ga-Cy3-CHP was analyzed by radioactive TLC (ITLC, 10 mM EDTA, pH 7), wherein Rf ⁶⁸Ga/EDTA = 1.0 and Rf ⁶⁸Ga-Cy3-CHP = 0.0. The results are shown in FIG. 3. There was no free radionuclide ⁶⁸Ga in the product, indicating that the probe ⁶⁸Ga-Cy3-CHP prepared in the present disclosure can achieve a high radiochemical yield without HPLC purification, with a specific activity range from 2.69 to 7.34 GBq/µmol.

### (2) In Vitro Stability Analysis

The ⁶⁸Ga-Cy3-CHP probe prepared in Example 1 was placed in 1x PBS and 10% fetal bovine serum (FBS) solutions, incubated at 37 °C for 2 h, and subjected to radioactive TLC detection every 30 min.

The detection results are shown in FIG. 4A and FIG. 4B. The results show no significant decrease in ⁶⁸Ga labeling after approximately 2 h, indicating that the probe prepared in the present disclosure exhibits good in vitro stability.

Probes prepared in other examples of the present disclosure possess radioactivity and in vitro stability comparable to those of ⁶⁸Ga-Cy3-CHP prepared in Example 1.

### Test Example 2: In Vivo Targeting of Denatured Collagen in Subcutaneous PDAC Tumor Lesions

In this test example, ⁶⁸Ga-Cy3-CHP prepared in Example 1 was used as a sample to evaluate the in vivo targeting specificity of the radiolabeled CHP-PET probe constructed in the present disclosure toward denatured collagen. The specific methods were as follows:
First, a mouse subcutaneous tumor model was constructed using the human pancreatic cancer cell line CFPAC-1 (3×10⁶ CFPAC-1 cells were injected into the right shoulder/back of 6-week-old female Balb/c-nu nude mice; experiments were conducted when the subcutaneous tumors reached 200 mm³). The mice were divided into three groups. In two of the groups, the subcutaneous tumor-bearing mice were injected via the tail vein with approximately 8.42 MBq of either the denatured collagen-targeting probe ⁶⁸Ga-Cy3-CHP or the non-targeting probe ⁶⁸Ga-Cy3-^{S}CHP (the probe prepared in Comparative Example 1). Another group of mice was pre-injected with a blocking dose of the non-radiolabeled precursor probe NOTA-Cy3-CHP before being injected with ⁶⁸Ga-Cy3-CHP (blocking group). Dynamic PET-CT imaging was performed for 1 h.

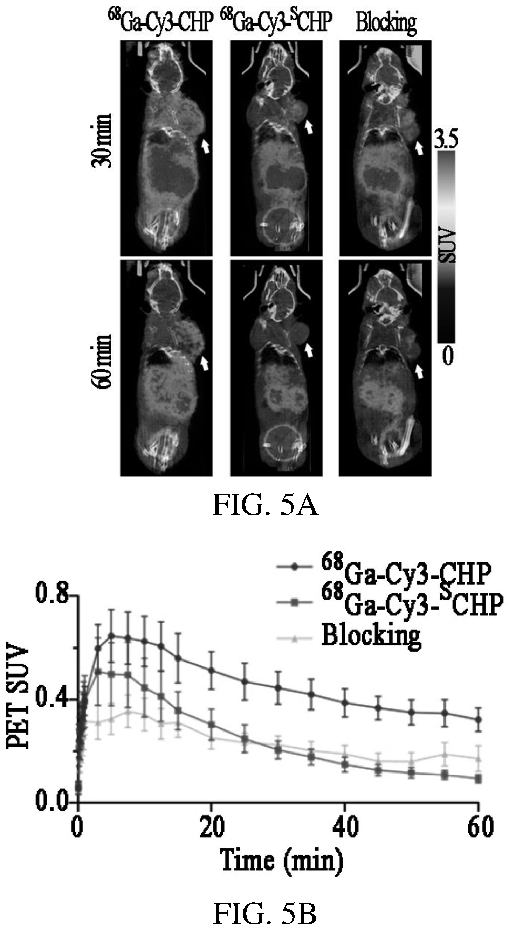

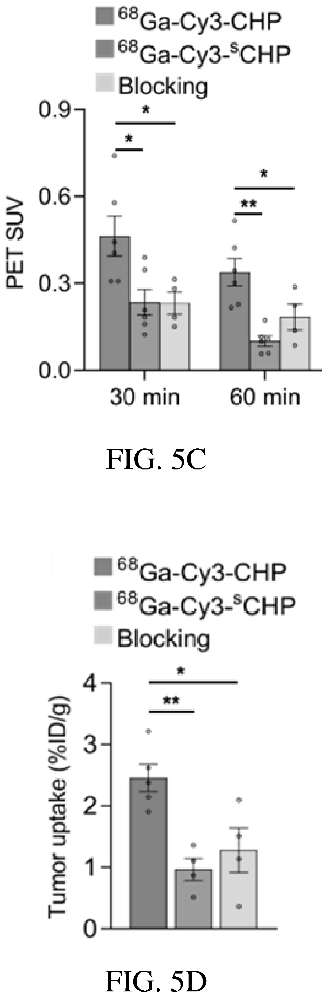

The detection results are shown in FIG. 5A-5D, where FIG. 5A shows representative PET images obtained at 30 min and 60 min during 1 h dynamic PET imaging after subcutaneous tumor-bearing mice were injected with ⁶⁸Ga-Cy3-CHP, or ⁶⁸Ga-Cy3-^{S}CHP, or subjected to blocking. FIG. 5B and FIG. 5C are the statistical results of tumor radioactivity quantification, respectively. FIG. 5D shows the results of ex vivo quantitative analysis of tumor uptake after 1 h of dynamic scanning. The results show that subcutaneous tumors can be clearly observed at 0.5 h and 1 h using ⁶⁸Ga-Cy3-CHP with the correct targeting sequence, providing a good tumor-to-background contrast; however, tumors are not observable in the ⁶⁸Ga-Cy3-^{S}CHP and blocking groups (as shown in FIG. 5A). The in vivo PET signals during the 1 h dynamic PET imaging are quantified as PET standardized uptake values (SUV). Compared with the rapid clearance of ⁶⁸Ga-Cy3-^{S}CHP and the sustained low signal in the blocking group, ⁶⁸Ga-Cy3-CHP washes out from the tumor slowly and maintains a significantly high signal throughout the dynamic imaging process (as shown in FIG. 5B and FIG. 5C). Quantification of the PET signals in the tumors at 30 min and 60 min shows statistically significant differences between the forward-sequence group and the scrambled-sequence or blocking groups.

To verify the PET imaging results, the mice were euthanized after the 1 h dynamic PET imaging, and the tumors were harvested for ex vivo biodistribution analysis, calculated as a percentage of the injected dose per gram (% ID/g). The results, shown in FIG. 5D, are consistent with the PET results, demonstrating that ⁶⁸Ga-Cy3-CHP has significantly higher tumor uptake. This further indicates that the denatured collagen-targeting probe of the present disclosure can be used for the in vivo detection of PDAC.

### Test Example 3: In Vivo Detection of Orthotopic Pancreatic Tumors in Deep Anatomical Locations

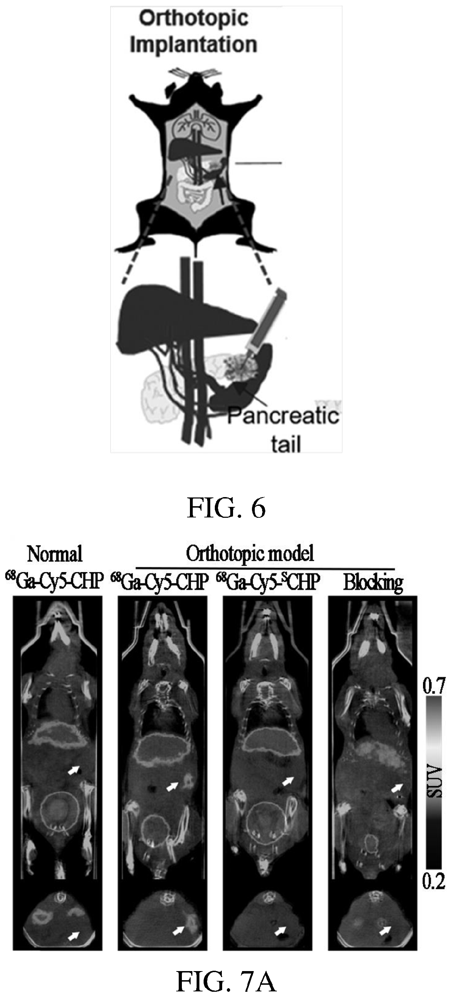

One reason PDAC is difficult to detect is its deep anatomical location. Therefore, this test example utilized an orthotopic tumor model using ⁶⁸Ga-Cy5-CHP prepared in Example 2 and ⁶⁸Ga-Cy5-^{S}CHP prepared in Comparative Example 2 as samples to evaluate the capability of the radiolabeled CHP-PET probe constructed in the present disclosure for in vivo detection of orthotopic pancreatic tumors in deep anatomical locations. The specific process included the following:
An orthotopic model was constructed by injecting 1x10⁶ human pancreatic cancer cells (CFPAC-1-LUC) into the tail of the pancreas of 6-week-old female Balb/c-nu nude mice, and the success of the model was confirmed via bioluminescence (as specifically shown in FIG. 6). Normal mice of the same age were injected via the tail vein with approximately 8.42 MBq of ⁶⁸Ga-Cy5-CHP; orthotopic tumor-bearing mice were injected via the tail vein with approximately 8.42 MBq of ⁶⁸Ga-Cy5-^{S}CHP (the probe from Comparative Example 2); and another group of mice was pre-injected with a blocking dose of the non-radiolabeled precursor probe NOTA-Cy5-CHP followed by the injection of ⁶⁸Ga-Cy5-CHP (blocking group). Static PET-CT imaging was performed for 2 h.

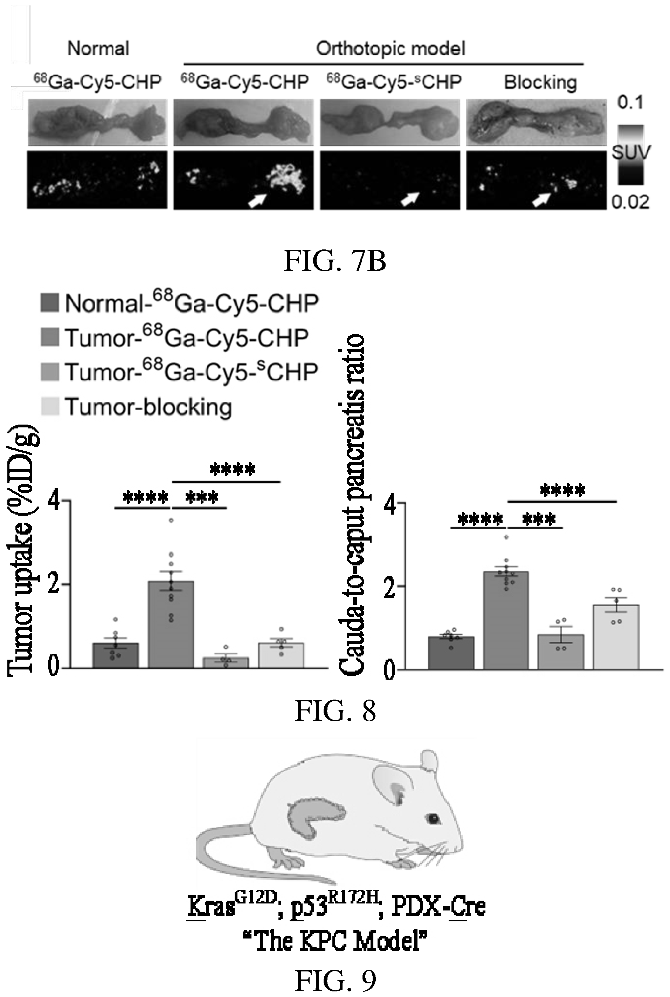

The detection results are shown in FIG. 7A and FIG. 7B. Consistent with the subcutaneous tumor results, the 2 h static PET-CT imaging shows that the ⁶⁸Ga-Cy5-CHP probe with the targeting sequence allows for in vivo visualization of pancreatic tumors. In contrast, no signal is observed in the pancreas of normal mice, or in model mice using the scrambled probe or the blocking group (as shown in FIG. 7A). After the static scan, the mice were sacrificed, and the pancreas was harvested for ex vivo PET imaging. The ex vivo PET results further supports the in vivo PET imaging findings (as shown in FIG. 7B).

Furthermore, since the orthotopic tumor model involves planting the tumor in the tail region of the pancreas, the harvested pancreas was divided into two parts: the pancreatic tail (tumor) and the pancreatic head. The radioactive uptake of both parts and the ratio of pancreatic tail to pancreatic head were calculated. The results are shown in FIG. 8, demonstrating significantly high uptake of ⁶⁸Ga-Cy5-CHP in the pancreatic tail tumor site. This further proves that the probe of the present disclosure can target denatured collagen in orthotopic abdominal pancreatic tumors in vivo and possesses the capability for abdominal detection.

### Test Example 4: In Vivo Visualization and Detection of Pancreatic Tumors

Since the probes target the ECM, there are significant differences between the tumor ECM induced after injecting human tumor cells, whether in subcutaneous or orthotopic tumor models, and the real tumor ECM. This test example utilized the transgenic KPC mouse model (LSL-Kras^{G12D/+}; LSL-Trp53^{R172H/+}; Pdx-1-Cre), which can spontaneously form tumors and possesses lesions and a microenvironment closer to human pancreatic cancer, as experimental material. The probes prepared in the examples of the present disclosure were used to detect PDAC lesions in the KPC model (>16 weeks of age) to further screen for preferred targeting probes.

### (1) ⁶⁸Ga-Cy5-CHP probe

In this section, the ⁶⁸Ga-Cy5-CHP probe prepared in Example 2 which has been proven to detect orthotopic abdominal pancreatic tumors in vivo was used as a detection marker to detect and label PDAC lesions in the KPC model (>16 weeks of age).

The KPC mouse model is the most commonly used genetically engineered mouse for studying the PDAC tumor microenvironment. This model contains conditional mutations of oncogene Kras activation and tumor suppressor gene TP53. The disease progression in KPC mice is very similar to that in humans (developing into PanIN at 8-10 weeks and PDAC at 16 weeks) and recapitulates many prominent clinical features (such as complications including cachexia, ascites, and biliary obstruction) and histopathological characteristics of human PDAC, as specifically shown in FIG. 9.

The specific experimental method was as follows: KPC mice and normal mice of the same age were taken, and approximately 10 MBq of ⁶⁸Ga-Cy5-CHP was injected into the tail vein of the mice, respectively. Static scans were performed for 2 h. After the static scans, the mice were sacrificed, the pancreas was harvested for ex vivo PET, and the radioactive biodistribution of all organs was determined.

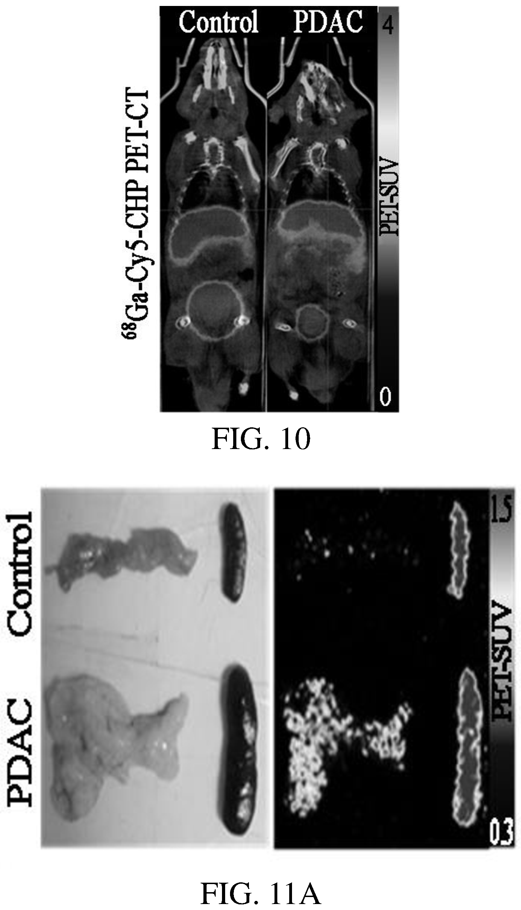

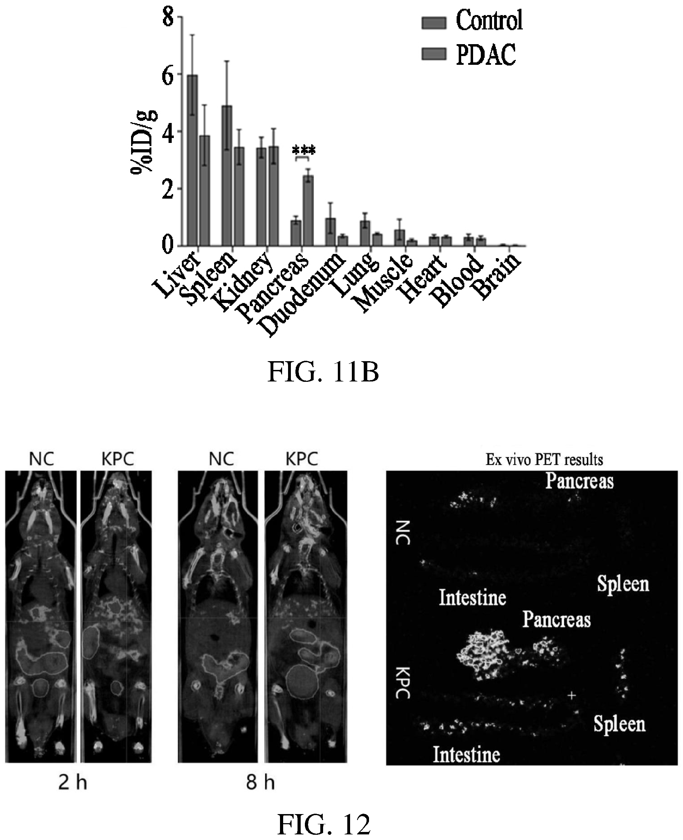

The detection results are shown in FIG. 10, FIG. 11A and FIG. 11B, where FIG. 10 is the 2 h static scan PET image of ⁶⁸Ga-Cy5-CHP, FIG. 11A is the ex vivo PET result of the pancreas, and FIG. 11B is the biodistribution result. Although the ex vivo PET and the biodistribution results of organs show significant enrichment of the probe at the tumor site of the KPC mice, unlike the orthotopic tumor-bearing mice where the tumor is concentrated in the tail of the pancreas (with a clear anatomical location and larger tumor), the tumors in KPC mice are scattered throughout the pancreas and are small tumors. The detection of the ⁶⁸Ga-Cy5-CHP probe is affected by organs around the pancreas such as the liver, spleen, and kidneys, which to some extent interferes with the in vivo visualization of PDAC.

The above results indicate that due to different nuclides, there will be certain differences in the biodistribution of the probes in vivo, thereby interfering with the signal-to-noise ratio of the probes in the target organs. If in vivo tumor visualization of KPC mice that more closely matches actual clinical features (tumors are scattered throughout the pancreas) is to be achieved, the probes need to be further improved and optimized.

### (2) ⁶⁴Cu-Cy5-CHP probe detection

In order to adjust the biodistribution of radionuclide-labeled molecular probes and thereby improve the in vivo imaging effect of pancreatic tumors, in this section, ⁶⁸Ga-Cy5-CHP was improved by using another metal nuclide, ⁶⁴Cu, i.e., ⁶⁴Cu-Cy5-CHP prepared in the above Example 3 was used for further detection. The specific method was as follows:
Approximately 10 MBq of ⁶⁴Cu-Cy5-CHP was injected into the tail vein of KPC mice (>16 weeks of age) and normal mice of the same age. Static scans were performed at multiple time points of 2 h, 4 h, 6 h, and 8 h. After 8 h, the pancreas was harvested for ex vivo PET.

The detection results are shown in FIG. 12, showing results similar to the ⁶⁸Ga-Cy5-CHP PET imaging results. Although ex vivo PET shows that the ⁶⁴Cu-Cy5-CHP probe is enriched at the tumor site, the strong signal from the abdominal intestines interferes with the in vivo visualization of PDAC.

### (3) Al¹⁸F-Cy5-CHP probe detection

In this section, the nuclide was changed to Al¹⁸F, i.e., the Al¹⁸F-Cy5-CHP probe prepared in Example 4 was used for in vivo imaging of pancreatic tumors. The specific method was as follows: approximately 10 MBq of Al¹⁸F-Cy5-CHP was injected into the tail vein of KPC mice (>16 weeks of age) and normal mice of the same age, and a 3 h static scan was performed.

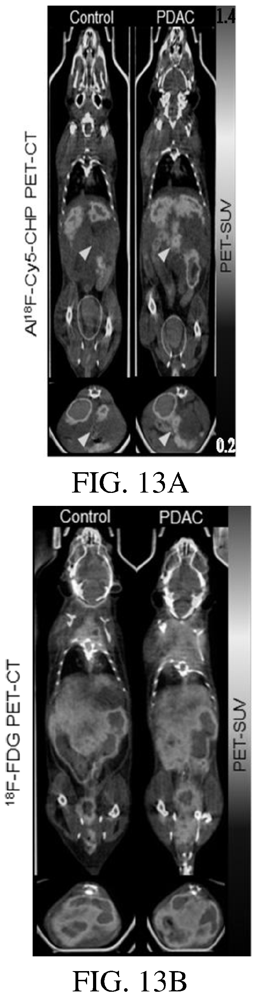

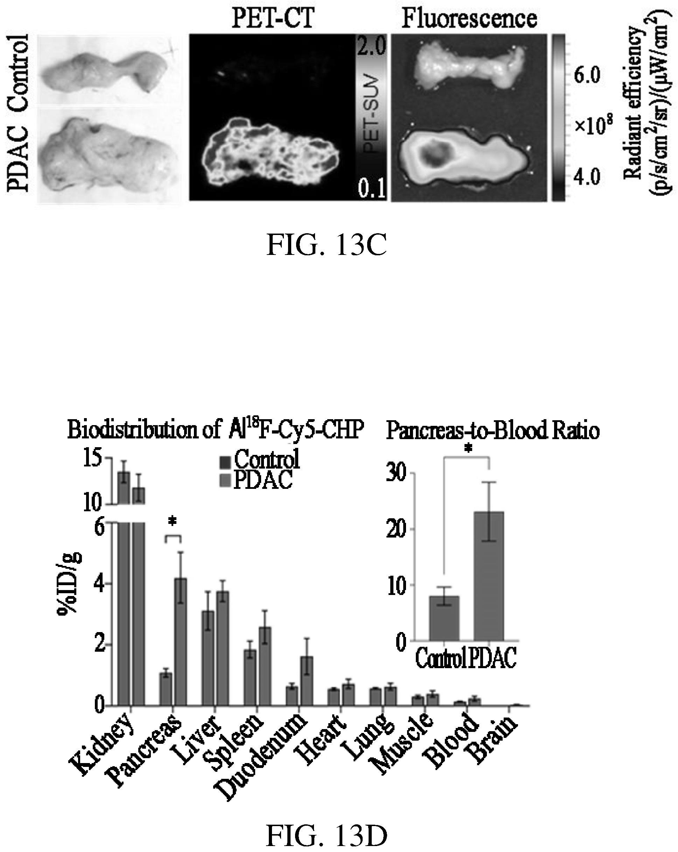

The detection results are shown in FIG. 13A to FIG.13D, where FIG. 13A is the 3 h static scan PET image of Al¹⁸F-Cy5-CHP, FIG. 13B is the 1 h static scan PET image of ¹⁸F-FDG in mice of the same age; FIG. 13C is the ex vivo PET result of the pancreas 3 h after injection of Al¹⁸F-Cy5-CHP, and FIG. 13D is the biodistribution result. The PET image results show that the uptake of Al¹⁸F-Cy5-CHP in the liver and spleen is significantly reduced, so that PDAC lesions in the KPC model can be accurately detected with high clarity and signal-to-noise ratio, and it is superior to traditional ¹⁸F-FDG. Ex vivo PET results and biodistribution further supports the PET data.

In summary, compared to the ⁶⁸Ga-Cy5-CHP probe and the ⁶⁴Cu-Cy5-CHP probe, the uptake of Al¹⁸F-Cy5-CHP in the liver is further reduced and the uptake in the pancreas is further increased, so that the abdominal pancreatic tumor can be clearly exposed in the PET image. The optimized Al¹⁸F-Cy5-CHP probe significantly improves the signal-to-noise ratio of pancreatic tumor detection, providing the possibility for achieving the detection of precancerous lesions.

### Test Example 5: In Vivo Detection of PanIN Lesions

In order to explore whether radionuclide-labeled CHP probes can detect pancreatic intraepithelial neoplasia (PanIN) lesions in vivo, this test example employed the Al¹⁸F-Cy5-CHP probe using younger KPC mice. Theoretically, 8-week-old KPC mice have not yet progressed to the PDAC stage. These KPC mice and normal mice of the same age were injected with approximately 10 MBq of Al¹⁸F-Cy5-CHP via the tail vein, followed by a 2 h static PET-CT scan.

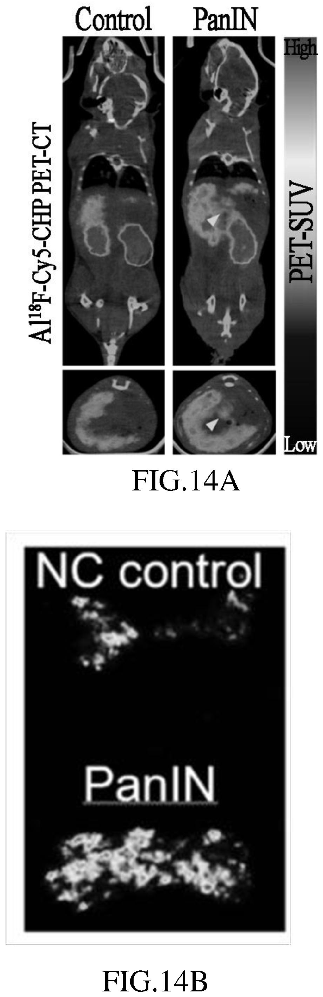

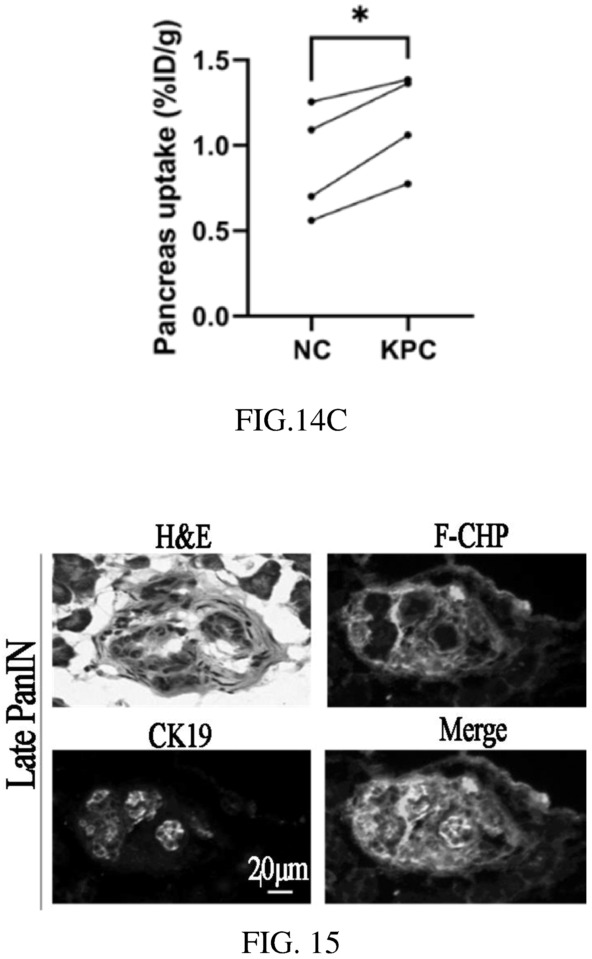

The detection results are shown in FIG. 14A to FIG.14C, where FIG. 14A is a representative PET image of the 2 h static scan of Al¹⁸F-Cy5-CHP, FIG. 14B is the ex vivo PET result of the pancreas, and FIG. 14C is the quantification of pancreatic radioactivity uptake. The results show high-signal points at the anatomical location of the pancreas in the abdomen. After imaging, the pancreas was harvested for ex vivo PET and its radioactivity was measured. The results confirm that there is indeed stronger Al¹⁸F-Cy5-CHP uptake in the KPC pancreas.

Furthermore, in this test example, frozen sections of the pancreas were prepared for HE staining and CHP counterstaining. The detection results are shown in FIG. 15. The results indicate that high-grade PanIN is present in the pancreas of low-week-old KPC mice, and a large amount of denatured collagen exist around the PanIN. This is because, during the evolution of PDAC, a large amount of unwound collagen already exists as early as the PanIN stage. Therefore, the pathological results confirm that the signal detected in vivo by Al¹⁸F-Cy5-CHP indeed originate from PanIN lesions. It can be seen that the Al¹⁸F-Cy5-CHP probe of the present disclosure has an excellent signal-to-noise ratio, enabling it to successfully detect such minute lesions as PanIN, providing a new tool for the early detection of PDAC.

In summary, from the partial detection results for pancreatic cancer, it can be seen that based on the CHP comprising a (GfO)ₙ sequence, the present disclosure has designed a class of radionuclide-labeled molecular probes (CHP-PET probes) that can specifically target structurally denatured collagen in the pathological ECM. The ⁶⁸Ga-labeled CHP-PET probe can detect denatured collagen in PDAC in subcutaneous and orthotopic mouse models with excellent specificity. Additionally, we utilized the transgenic KPC mouse model that spontaneously forms PDAC. This model recapitulates the different stages of PDAC progression (including PanIN) and possesses a tumor microenvironment closer to human PDAC, thereby providing results that are more consistent with actual clinical applications. Regrettably, due to unsuitable biodistribution, neither the ⁶⁸Ga- nor the ⁶⁴Cu-labeled CHP-PET probes could achieve in vivo visualization of pancreatic tumors in KPC mice. To improve the signal-to-noise ratio of the CHP-PET probe at the site of pancreatic tumor lesions, the present disclosure optimized the CHP-PET probe. The optimized Al¹⁸F-labeled CHP-PET probe can not only detect PDAC in the KPC model but also detect PanIN lesions with excellent clarity and signal-to-noise ratio, which is superior to traditional FDG PET/CT imaging, providing a powerful new tool for the early detection of pancreatic cancer.

### Test Example 6: Detection and Staging of Collagen Remodeling During the Progression of Idiopathic Pulmonary Fibrosis

To further verify the disease detection versatility of the CHP-PET probe, this test example used a bleomycin-induced pulmonary fibrosis mouse model to explore the potential of the CHP-PET probe for detecting pulmonary fibrosis. The specific method was as follows:
Bleomycin (BM) was dissolved in PBS and administered in a single dose of 5.0 mg/kg to each male C57BL/6J mouse (6-8 weeks old); and the control group received no treatment. After bleomycin inhalation, the animals were rotated up and down to distribute the drug solution evenly throughout the lungs. The mice were observed for transient respiratory distress during the intranasal instillation process. The mice were placed in cages and, after their breathing stabilized, returned to the SPF-grade animal room for rearing. On day 28 after drug-induced pulmonary fibrosis, mice were intravenously injected with approximately 9.67 MBq of ⁶⁸Ga-Cy5-CHP or ⁶⁸Ga-Cy5-^{S}CHP.

The PET images at 2 hours post-injection are shown in FIG. 16A, demonstrating that ⁶⁸Ga-Cy5-CHP specifically accumulates in the fibrotic lungs of BM-treated mice at 28 days, but not in the healthy lungs of control mice, whereas the non-targeting ⁶⁸Ga-Cy5-^{S}CHP does not preferentially enter the lungs of BM-treated mice. Immediately after the PET/CT scan, the lungs were collected and subjected to radioactivity measurement. The quantitative results further supports the PET imaging (as shown in FIG. 16B). Subsequently, PET-CT imaging was performed for the 7-day inflammatory stage after drug induction. The imaging and quantitative results show that the uptake of ⁶⁸Ga-Cy5-CHP in the lungs is also significantly higher than that of the normal control (as shown in FIG. 16C and FIG. 16D). It can be seen that ⁶⁸Ga-Cy5-CHP can detect lung lesions in vivo and has the potential for non-invasive detection of early pulmonary fibrosis.

The above results show that, in addition to detecting pancreatic cancer, ⁶⁸Ga-Cy5-CHP can detect and stage collagen remodeling during the progression of idiopathic pulmonary fibrosis. This result proves that due to the conservation of structurally denatured collagen in various fibrotic diseases, the CHP-PET probe developed in the present disclosure for detecting collagen structural denaturation can serve as a platform technology for the detection of other fibrotic diseases.

### Test Example 7: CHP Hybridization Reveals Response of Mice to Anti-Fibrotic Therapy

Based on the static fact that the increase or decrease of ECM collagen only reflects ECM deposition or regression, the production of structurally denatured collagen is related to both collagen generation and collagen degradation. Therefore, detecting denatured collagen can also characterize the dynamic changes of ECM remodeling during disease progression and explore the underlying mechanisms. Since there are currently no therapies capable of preventing or reversing organ fibrosis, the lack of effective drug efficacy detection is considered a major factor limiting the development of anti-fibrotic therapies. Thus, the detection of denatured collagen can assist in the research of disease progression and drug development.

This test example utilized the ⁶⁸Ga-Cy5-CHP probe prepared in Example 2 to characterize the in vivo response of mice to two different anti-fibrotic treatments, further proving that the probe developed in the present disclosure can be used not only for disease detection imaging but also to reflect the dynamic changes of ECM remodeling, guiding mechanistic studies of disease progression and efficacy studies in drug development. The specific characterization methods of this test example were as follows:

### (1) Efficacy testing for Pirfenidone (PFD)

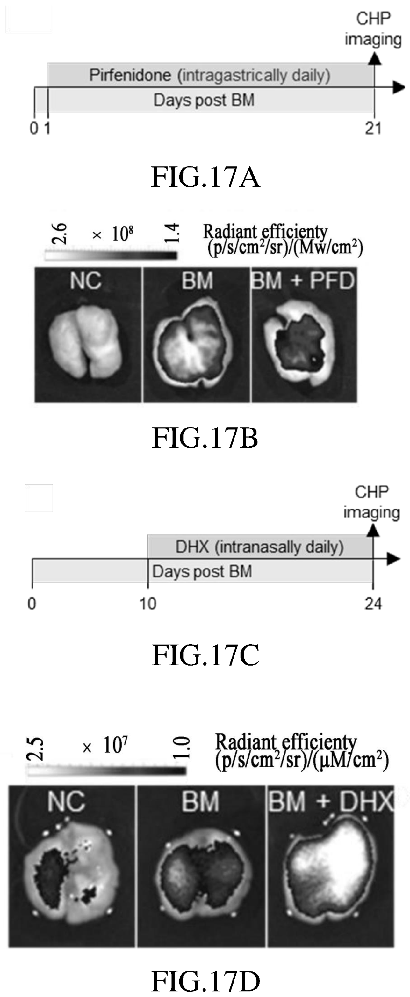

As one of the two conditionally recommended therapies for IPF in clinical practice, PFD exerts anti-fibrotic effects by inhibiting fibroblast proliferation and differentiation, in addition to inhibiting TGF-β1 production and collagen synthesis. PFD inhibits the synthesis and degradation of collagen. Therefore, this test example anticipated that the lungs of BM-injured mice treated with PFD should have less denatured collagen. A schematic diagram of the specific PFD treatment method is shown in FIG. 17A. PFD treatment was continuously administered one day after bleomycin modeling. On the 21st day after modeling, ⁶⁸Ga-Cy5-CHP was injected into the tail vein of the mice, and the lungs were harvested for fluorescence imaging at 4 h.

The results show that, compared with the untreated BM control, the PFD treatment group significantly reduces the lung uptake of the probe in vivo (as shown in FIG. 17B), indicating that the hybridization of CHP with denatured collagen caused by pulmonary fibrosis remodeling can reflect the response of mice to anti-fibrotic treatment.

### (2) Study on Dihydrexidine (DHX) treatment pathway

In addition to inhibiting inflammation and collagen synthesis, there are new strategies targeting the collagen decomposition pathway to promote the absorption of ECM in IPF. For example, the agonistic effect of dihydrexidine (DHX) on dopamine receptor D1 in lung fibroblasts has been shown to accelerate the clearance of excessive ECM in mouse pulmonary fibrosis tissue by up-regulating Cathepsin K. Therefore, DHX promotes the degradation of collagen to achieve the effect of alleviating fibrosis.

It was first hypothesized that fibrotic lungs treated with DHX would show less collagen accumulation but higher levels of collagen degradation and denaturation compared to the untreated BM control group. Furthermore, this test example confirmed this hypothesis with animal experiments. A schematic diagram of the specific DHX treatment method is shown in FIG. 17C. DHX treatment was continuously administered 10 days after bleomycin modeling. On the 24th day after modeling, ⁶⁸Ga-Cy5-CHP was injected into the tail vein of the mice, and the lungs were harvested for fluorescence imaging at 4 h.

The detection results are shown in FIG. 17D, demonstrating that the uptake of the probe in the lungs of the DHX treatment group is significantly higher than that of the untreated BM disease group. The above results indicate that collagen hybridization can reveal the subtle differences between different therapeutic pathways (inhibiting synthesis versus promoting degradation) related to collagen metabolism.

From the detection results of the above Test Examples 1-7, it can be seen that the radionuclide-labeled molecular probes designed in the present disclosure, which specifically target structurally denatured collagen in pathological ECM, provide a new and powerful tool for the early detection of pancreatic cancer. Moreover, due to the conservation of ECM proteins, they can serve as a platform technology applied in the detection and treatment of various other diseases characterized by ECM deposition. Simultaneously, because denatured collagen reflects the dynamic changes of ECM remodeling during disease progression, the probes developed in the present disclosure can be used not only for disease detection imaging but also for guiding mechanistic research on disease progression and efficacy studies in drug development.

### Test Example 8: Evaluation of Chemical Stability

In nuclide labeling reaction systems, only a trace amount of nuclide is present (for example, 666 MBq of ⁶⁸Ga is only 0.00648 nmol; 2.5 GBq of ¹⁸F is only 0.03944 nmol). Consequently, the precursor (50 nmol) added in each reaction is in vast excess compared to the nuclide atoms. Therefore, if the probe molecular structure remains stable during the radiolabeling process, a sufficient amount of precursor molecules should remain in the reaction solution after labeling. This test example performed radio-HPLC analysis on the reaction solutions after radiolabeling for the different probes prepared in Examples 5-7 and Comparative Examples 4 and 6, and compared the resulting profiles with the HPLC profiles of their corresponding probe precursors to determine stability.

Judgment Criterion: If a peak appears in the labeled reaction solution at the same retention time as the corresponding probe precursor, it can be concluded that excess precursor exists in the reaction solution, indicating that the probe precursor structure is stable and has not undergone radiation degradation.

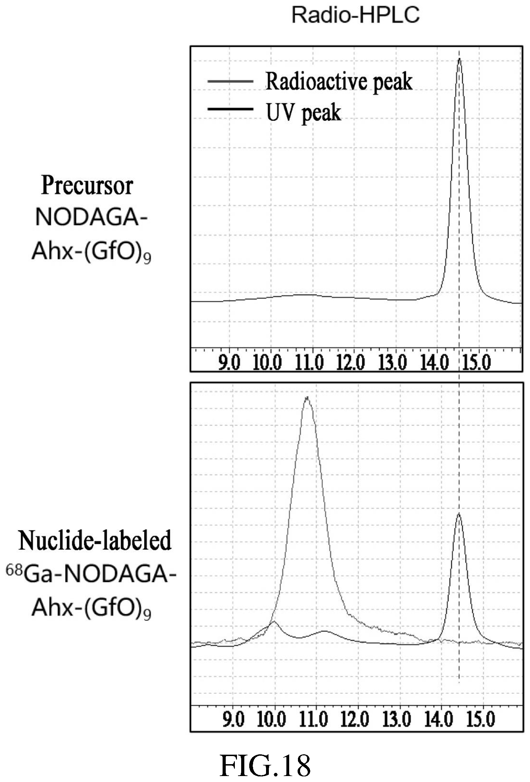

The HPLC profiles of the reaction solution after labeling and its probe precursor during the preparation of the radiolabeled collagen hybridizing peptide ⁶⁸Ga-NODAGA-Ahx-(GfO)₉ are shown in FIG. 18. The results show that a sufficient amount of precursor molecules remain after ⁶⁸Ga labeling, and there is only one radioactive peak, indicating that this probe structure has good stability and does not undergo radiation degradation.

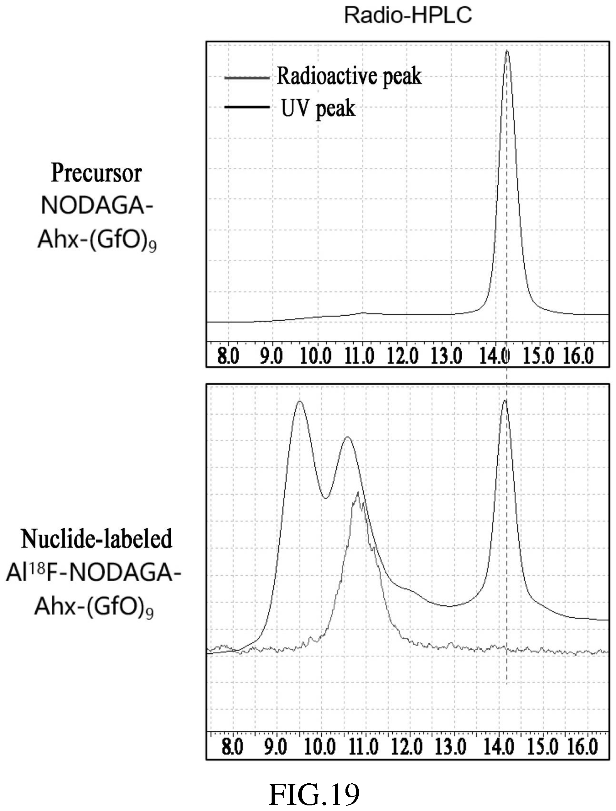

The HPLC profiles of the reaction solution after labeling and its probe precursor during the preparation of the radiolabeled collagen hybridizing peptide Al¹⁸F-NODAGA-Ahx-(GfO)₉ are shown in FIG. 19. The results indicate that this probe is very stable during the Al¹⁸F labeling process, with only one radioactive peak. Furthermore, excess unchelated and structurally intact precursor exists in the product solution, demonstrating that the probe precursor structure has good stability and does not undergo radiation degradation.

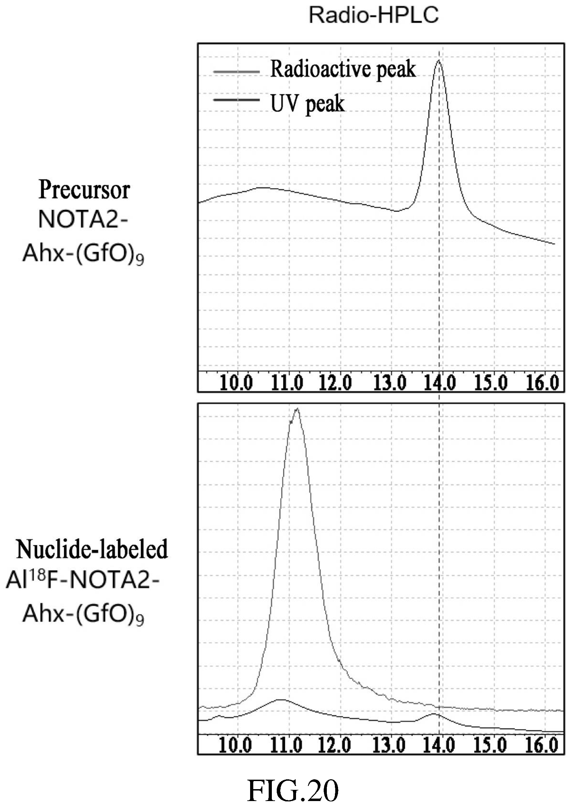

The HPLC profiles of the reaction solution after labeling and its probe precursor during the preparation of the radiolabeled collagen hybridizing peptide Al¹⁸F-NOTA2-Ahx-(GfO)₉ are shown in FIG. 20. The results show only one radioactive peak and the presence of excess unchelated, structurally intact precursor, proving that this probe structure is stable during the Al¹⁸F labeling process and does not undergo radiation degradation.

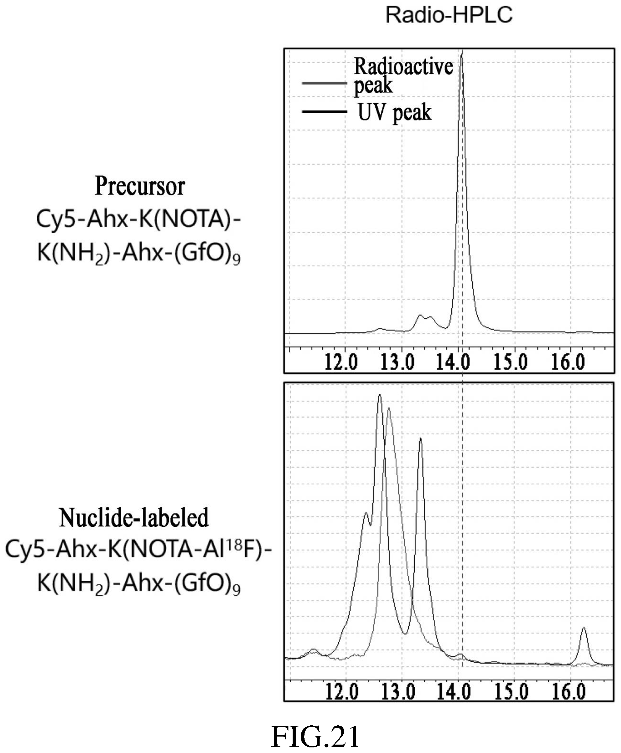

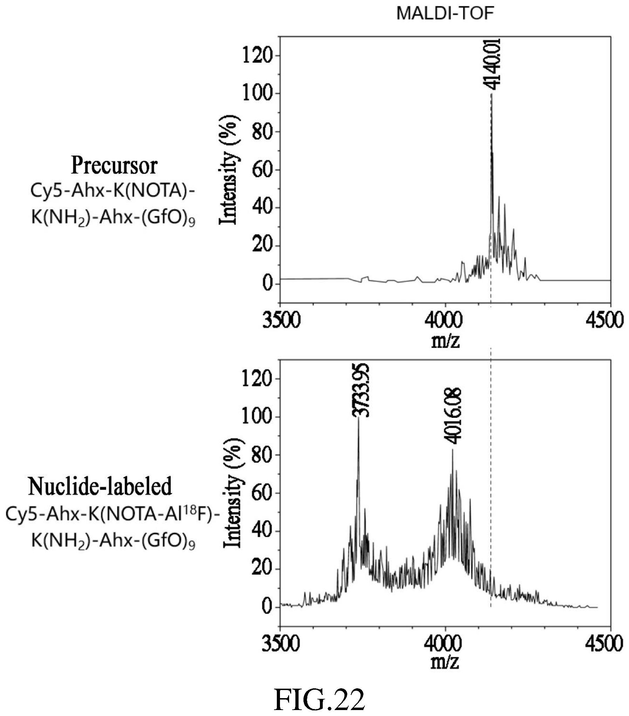

The HPLC profiles before and after nuclide labeling for the fluorescence-radiolabeled collagen hybridizing peptide Cy5-Ahx-K(NOTA-Al¹⁸F)-K(NH₂)-Ahx-(GfO)₉ prepared in Comparative Example 4 are shown in FIG. 21. The results show that no UV peak consistent with the precursor's retention time exists in the solution after labeling (the position indicated by the red line), indicating that the excess probe precursor compound in the reaction solution is decomposed. Furthermore, matrix-assisted laser desorption/Ionization time-of-Flight mass spectrometry (MALDI-TOF) analysis was performed on the reaction solution to detect molecular weights. The results, shown in FIG. 22, reveal that the molecular weights of substances in the reaction solution (3733.95 Da, 4016.08 Da) are all smaller than that of the probe precursor (4140.01 Da), confirming that this precursor molecule is unstable during the Al¹⁸F labeling process and undergo radiation decomposition.

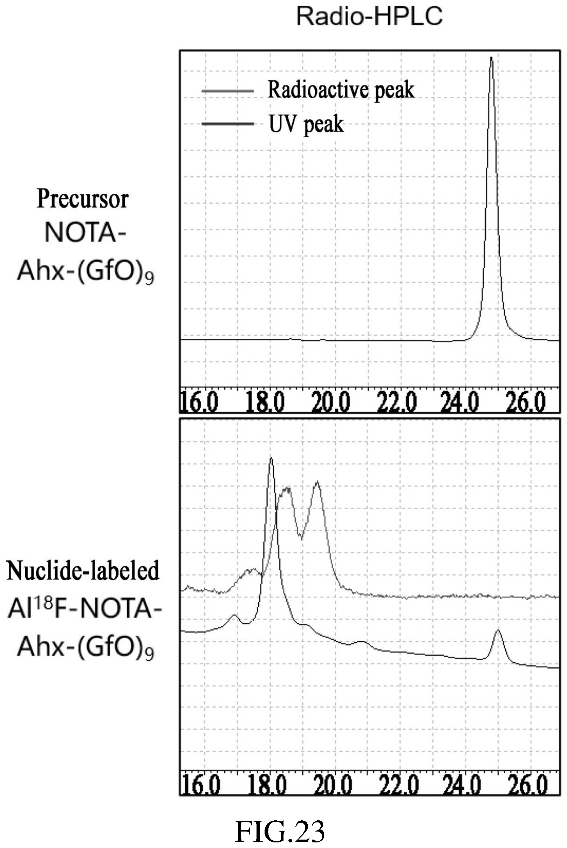

The HPLC profiles of the reaction solution after labeling and its probe precursor during the preparation of the radiolabeled collagen hybridizing peptide Al¹⁸F-NOTA-Ahx-(GfO)₉ are shown in FIG. 23. The results show multiple radioactive peaks in the solution after labeling (pink line, 17.5-20.5 min), indicating the presence of various radiolabeled substances. This reveals that the structure is unstable during the Al¹⁸F labeling process and undergo radiation decomposition.

The above results demonstrate that the radiolabeled collagen hybridizing peptides of the present disclosure maintain structural stability during the radiolabeling process. The probe precursors undergo almost no radiation decomposition after nuclide labeling, which helps to improve synthesis stability, radiochemical labeling yield, and purity.

### Test Example 9: Evaluation of Biodistribution Characteristics

In order to intuitively evaluate the in vivo targeting specificity for denatured collagen and the in vivo biodistribution effects of the various constructed CHP-PET probes, this test example used the human pancreatic cancer cell line CFPAC-1 to construct a mouse subcutaneous tumor model. Subsequently, approximately 8.42 MBq of the collagen hybridizing peptide probes prepared in the above Examples 5-7 and Comparative Examples 3-6 were injected via the tail vein, and PET-CT imaging was performed at designated times.

The specific construction method for the mouse subcutaneous tumor model was as follows: 3 × 10⁶ CFPAC-1 cells were injected into the right shoulder/back of 6-week-old female Balb/c-nu nude mice, and subsequent experiments were conducted when the subcutaneous tumors reached 200 mm³.

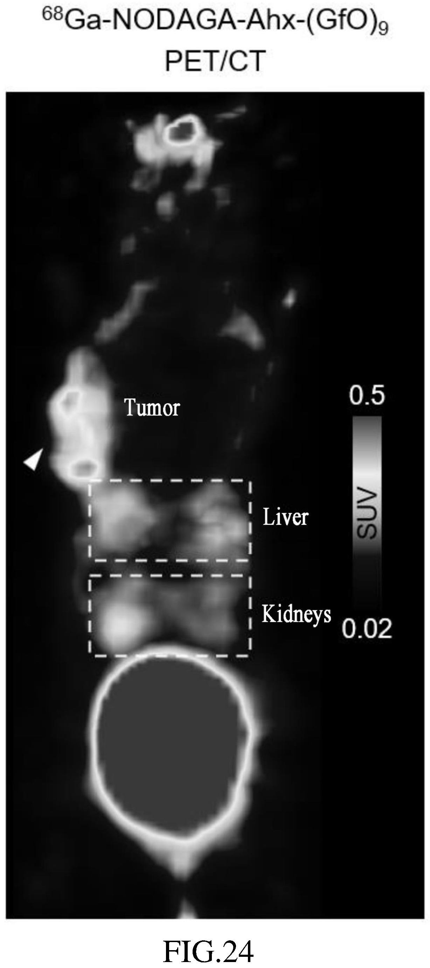

The PET/CT results of the radiolabeled collagen hybridizing peptide ⁶⁸Ga-NODAGA-Ahx-(GfO)₉ in subcutaneous pancreatic cancer tumor-bearing mice are shown in FIG. 24. The results show that the probe possesses excellent biodistribution effects in subcutaneous pancreatic cancer tumor-bearing mice, specifically manifested as significant signals in the subcutaneous tumor target organs, while uptake in non-target metabolic organs such as the liver and kidneys is low. At 1 h imaging after tail vein injection, most of the probe have already been excreted from the kidneys into the bladder. The overall low background signal result in a high signal-to-noise ratio at the tumor site. The excellent biodistribution characteristics of this probe provide the possibility for clinical translation.

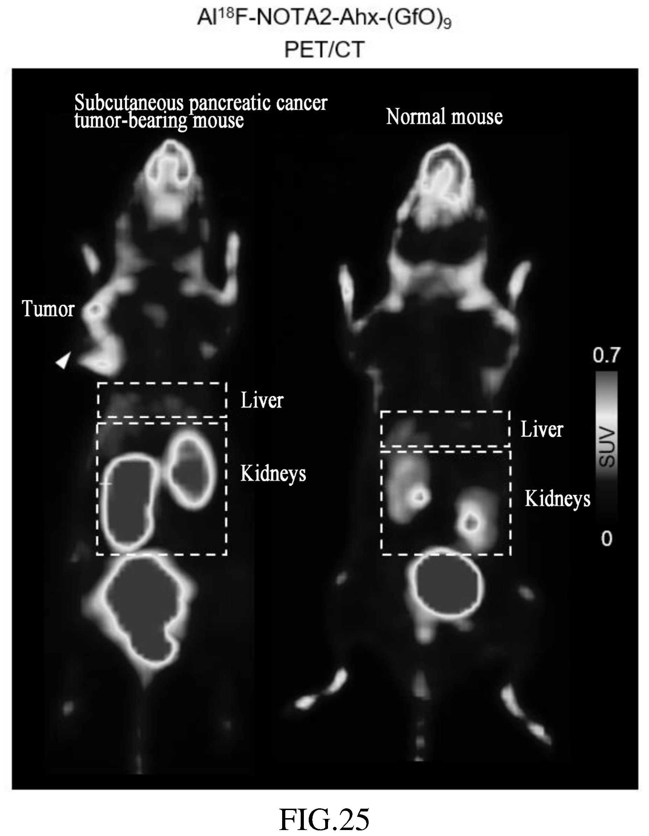

The 1 h PET/CT results of the radiolabeled collagen hybridizing peptide Al¹⁸F-NOTA2-Ahx-(GfO)₉ in subcutaneous pancreatic cancer tumor-bearing mice and normal mice are shown in FIG. 25. The results show that the biodistribution effect of this probe in subcutaneous pancreatic cancer tumor-bearing mice or normal mice is similar to that of the probe ⁶⁸Ga-NODAGA-Ahx-(GfO)₉ prepared in Example 1, also exhibiting an excellent tumor signal-to-noise ratio. In particular, it has extremely low signals in important non-target organs such as the liver and heart, which helps to improve the accuracy of in vivo imaging.

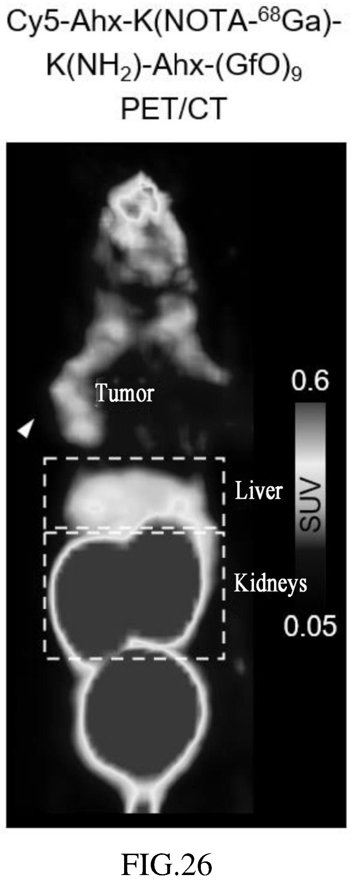

The PET/CT results of Cy5-Ahx-K(NOTA-⁶⁸Ga)-K(NH₂)-Ahx-(GfO)₉ prepared in Comparative Example 3 in subcutaneous pancreatic cancer tumor-bearing mice are shown in FIG. 26. The results show that 1 h after injection into the mice, strong signals are detected in the liver, kidney, and pancreatic positions, especially in the kidneys, which causes strong signal interference for the in vivo imaging of lesions in the pancreatic region.

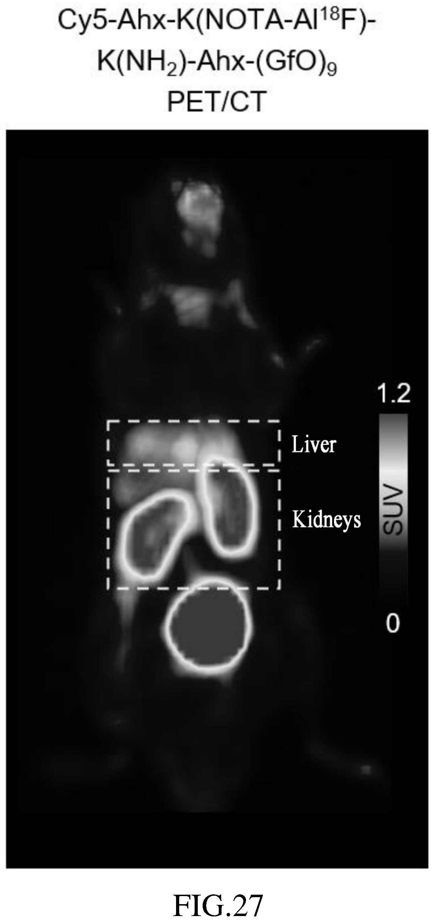

The 1 h PET/CT results of Cy5-Ahx-K(NOTA-Al¹⁸F)-K(NH₂)-Ahx-(GfO)₉ prepared in Comparative Example 4 in mice are shown in FIG. 27. The results show that although the probe exhibits low liver uptake low, its renal uptake is strong, which still interferes with the imaging of organs around the kidneys, such as the pancreas.

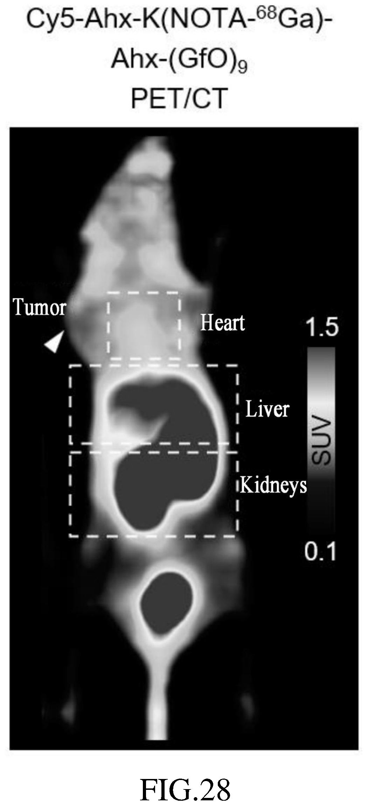

The 1 h PET/CT results of Cy5-Ahx-K(NOTA-⁶⁸Ga)-Ahx-(GfO)₉ prepared in Comparative Example 5 in subcutaneous pancreatic cancer tumor-bearing mice are shown in FIG. 28. The difference between this probe and the probe in Comparative Example 1 lies in the removal of the lysine [K(NH₂)] residue. The detection results show that the biodistribution of this probe changes significantly, with extremely strong uptake in the liver, kidneys, etc. In addition, there is also strong uptake in non-target organs such as the heart. High background noise makes the tumor signal almost invisible.

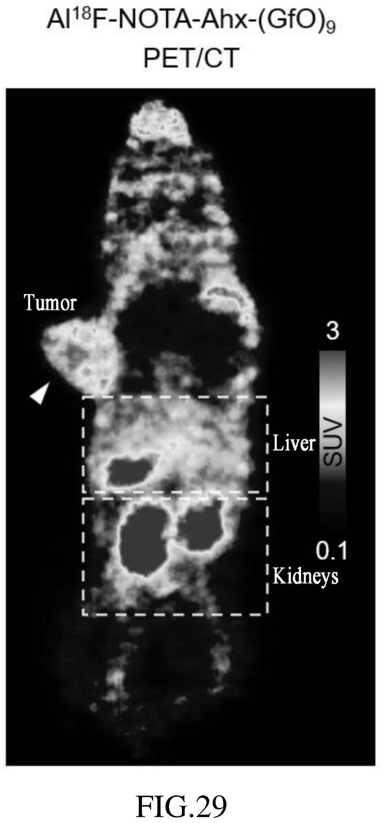

The 1 h PET/CT results of the radiolabeled collagen hybridizing peptide Al¹⁸F-NOTA-Ahx-(GfO)₉ prepared in Comparative Example 6 in subcutaneous pancreatic cancer tumor-bearing mice are shown in FIG. 29. The difference between this probe and the probe in Comparative Example 4 lies in the removal of the fluorescent dye moiety and other redundant amino acids. The results show that although this probe has signal accumulation in subcutaneous tumors, it also exhibits extremely strong uptake in the liver, kidneys, etc. Its biodistribution characteristics were worse compared to the probe in Comparative Example 4.

Furthermore, by comparing the probes prepared in Comparative Examples 5 and 6, it can be analyzed and concluded that simple structural deletions based on the original explored structure cannot necessarily improve the radiolabeling stability or the biodistribution characteristics of the probe for the preparation of probes used for PET in vivo imaging or even clinical research. Even with subtle differences in functional groups in the probe structure, the in vivo biodistribution characteristics can differ vastly. Currently, there is no method to accurately predict the relationship between chemical structure and biodistribution characteristics; therefore, systematic optimization and verification of different probe structures are required.

The above results show that the radiolabeled collagen hybridizing peptides prepared in the present disclosure possess excellent biodistribution characteristics with high uptake in target organs such as tumors and low uptake in non-target organs such as the liver and kidneys, possessing important clinical translation value.

From the above Test Examples 8-9, it can be seen that through optimized design of the structure of radiolabeled collagen hybridizing peptides, the present disclosure further screens for radiolabeled collagen hybridizing peptides that not only possess radiolabeling stability characteristics but also possess excellent biodistribution characteristics with high uptake in target organs such as tumors and low uptake in non-target organs such as the liver and kidneys.

The embodiments of the present disclosure have been described in detail above, but the present disclosure is not limited to the above embodiments. Within the scope of knowledge possessed by those of ordinary skill in the art, various changes can also be made without departing from the purpose of the present disclosure. In addition, where there is no conflict, the embodiments of the present disclosure and the features in the embodiments can be combined with each other.

## Claims

1. A radiolabeled collagen hybridizing peptide, comprising:
(a) a radionuclide-chelator complex bound via a coordination bond; and
(b) a collagen hybridizing peptide (CHP);
wherein the radionuclide-chelator complex and the CHP is connected through a linker, the linker comprises at least one selected from the group consisting of a lysine residue, an oligoglycine residue, 6-aminohexanoic acid, and polyethylene glycol (PEG); and
wherein an amino acid sequence of the CHP is (GfO)ₙ or (GPO)ₙ, where n is a positive integer between 6 and 10.

2. The radiolabeled CHP according to claim 1, wherein the radionuclide of the radionuclide-chelator complex is any one selected from the group consisting of Al¹⁸F, ⁶⁴Cu, ⁶⁷Cu, ⁶⁷Ga, ⁶⁸Ga, ⁹⁹mTc, ⁸⁹Zr, ¹¹¹In, ¹⁷⁷Lu, ¹⁸⁶Re, and ²²⁵Ac.

3. The radiolabeled CHP according to claim 1, wherein the chelator of the radionuclide-chelator complex is any one selected from the group consisting of NOTA, NODAGA, HYNIC, DOTA, DTPA, and derivatives thereof;
wherein the derivatives of NOTA comprise NOTA-NH₂ or p-SCN-Bn-NOTA;
wherein the NOTA-NH₂ has a structure represented by Formula (a-1): 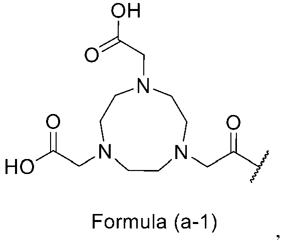 wherein the p-SCN-Bn-NOTA has a structure represented by Formula (a-2): 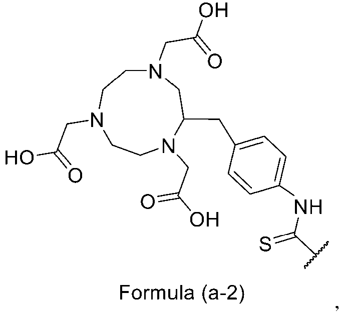 wherein NODAGA has a structure represented as follows: 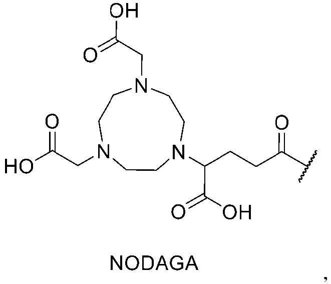
wherein the DOTA has a structure represented as follows:

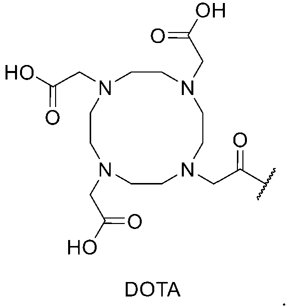

4. The radiolabeled CHP according to claim 1, wherein the radiolabeled CHP further comprises a fluorescent dye;
wherein the fluorescent dye is connected to the linker through an amino acid or an amino acid derivative; and
wherein the fluorescent dye is any one selected from the group consisting of a cyanine dye, rhodamine, BODIPY, FITC, erythrosin, phthalocyanine, phycocyanin, phycoerythrin, and an Alexa Fluor dye.

5. The radiolabeled CHP according to any one of claims 1 to 4, wherein:
when the linker of the radiolabeled CHP comprises a lysine residue and 6-aminohexanoic acid, the radiolabeled CHP is a compound represented by any one of Formulae (I-1) to Formulae (I-4): 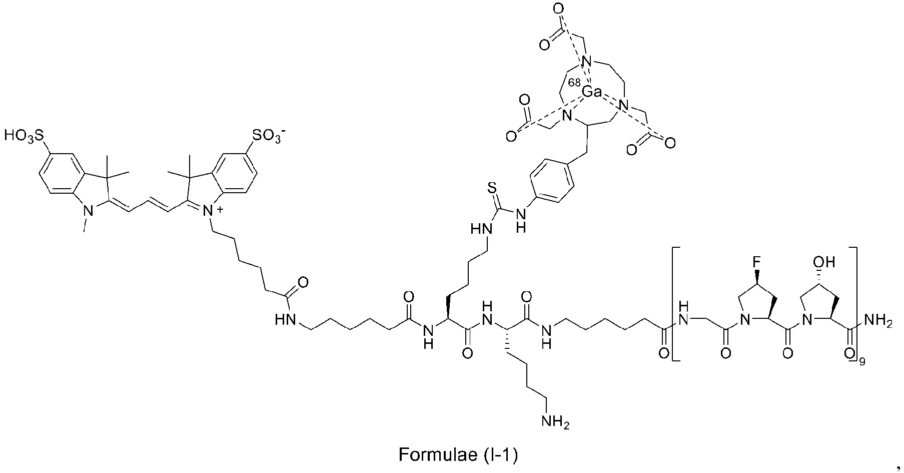 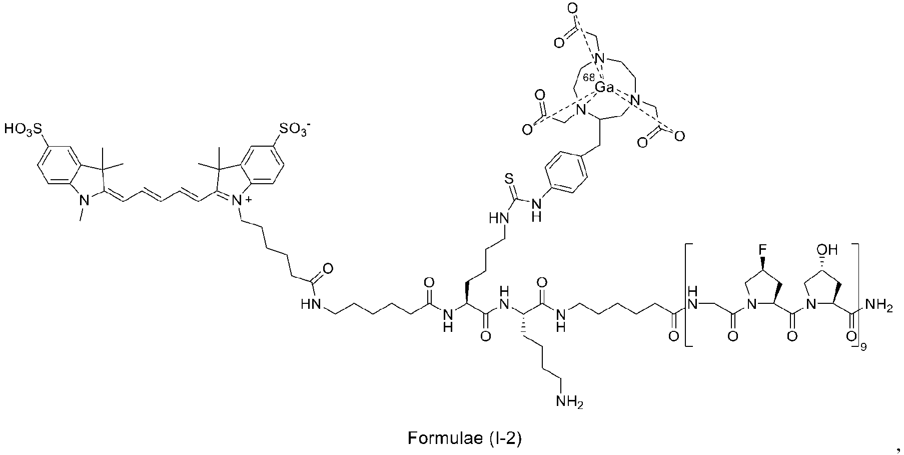 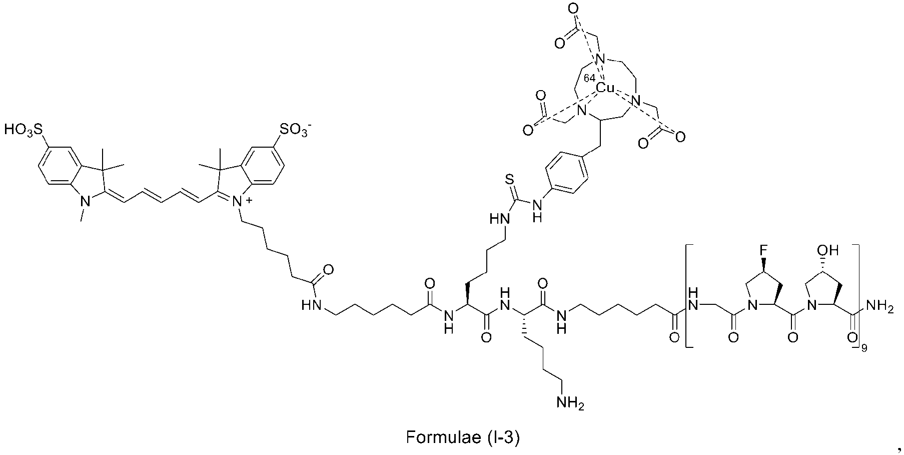 and 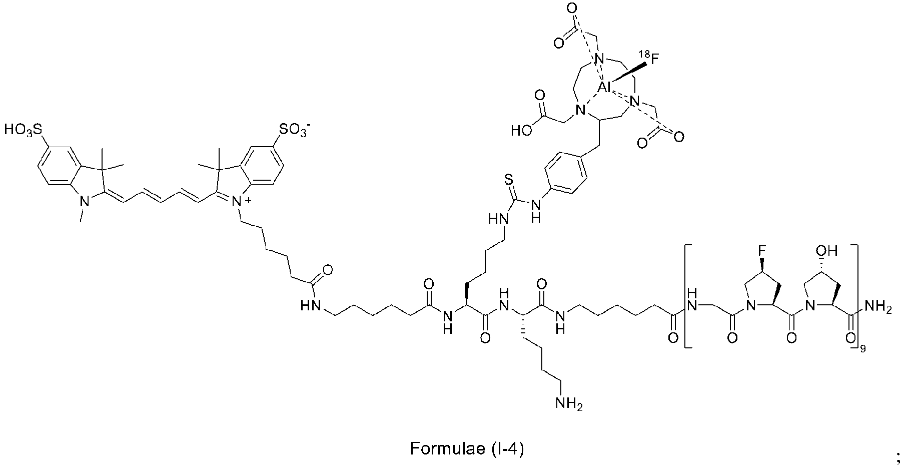
or, when the linker of the radiolabeled CHP is 6-aminohexanoic acid, the radiolabeled CHP is a compound represented by any one of Formulae (II-1) to Formulae (II-4): 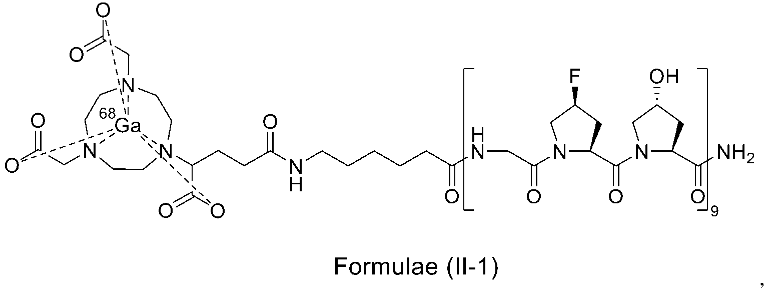 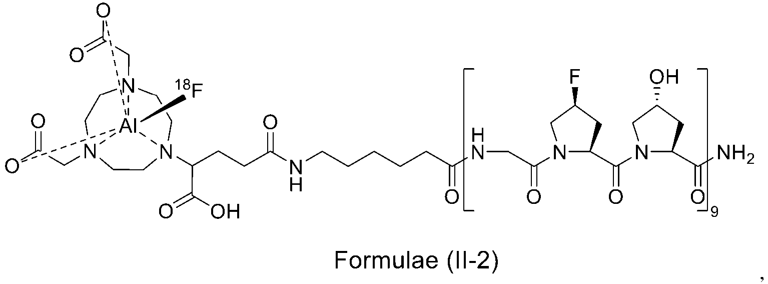 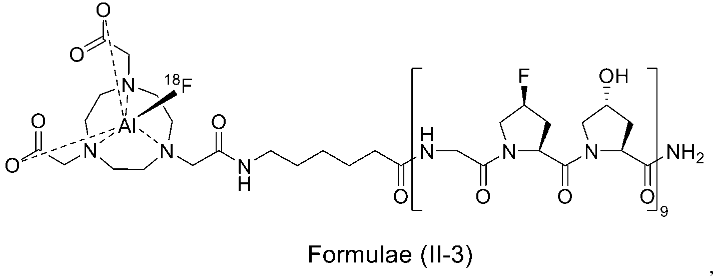 and

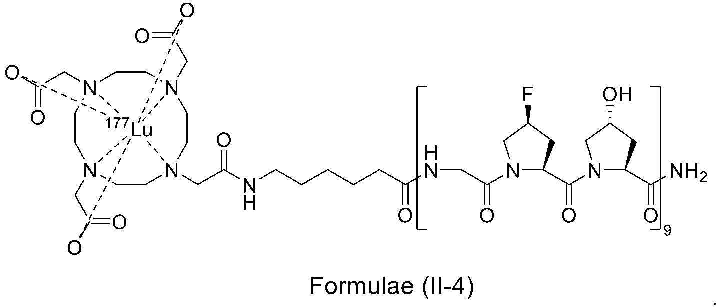

6. A method for preparing the radiolabeled CHP according to any one of claims 1 to 5, comprising:
obtaining a precursor probe containing the chelator and the CHP by a solid-phase peptide synthesis method; and labeling the precursor probe with the radionuclide to obtain the radiolabeled CHP.

7. A conjugate, comprising the radiolabeled CHP according to any one of claims 1 to 5, and a coupling moiety;
wherein the coupling moiety comprises at least one selected from the group consisting of a protein, a drug, and a detectable marker.

8. An imaging agent, comprising the radiolabeled CHP according to any one of claims 1 to 5.

9. Use of the radiolabeled CHP according to any one of claims 1 to 5 in any one of the following:
A) preparation of a product for specifically targeting a structurally denatured collagen;
B) preparation of a product for diagnosis or lesion imaging of cancer **characterized by** fibrotic lesions;
C) preparation of a product for detecting an organ fibrotic disease; or
D) evaluation or screening of an anti-pancreatic ductal adenocarcinoma drug or an anti-fibrotic drug.

10. A kit for imaging tumors or organ fibrosis, comprising the radiolabeled CHP according to any one of claims 1 to 5.

11. A method for treating or diagnosing a disease associated with organ fibrosis, comprising administering to a subject the radiolabeled CHP according to any one of claims 1 to 5, the conjugate according to claim 7, or the imaging agent according to claim 8.
